(19) Europäisches Patentamt
European Patent Office
Office européen des brevets

(11) **EP 4 237 399 B1**

(12) **EUROPEAN PATENT SPECIFICATION**

(45) Date of publication and mention
of the grant of the patent:
**21.08.2024 Bulletin 2024/34**

(21) Application number: **21794889.2**

(22) Date of filing: **28.10.2021**

(51) International Patent Classification (IPC):
*C07C 41/36* (2006.01)  *C07C 41/42* (2006.01)
*C07C 43/13* (2006.01)

(52) Cooperative Patent Classification (CPC):
(C-Sets available)
**C07C 43/13; C07C 41/36; C07C 41/42**  (Cont.)

(86) International application number:
**PCT/EP2021/079975**

(87) International publication number:
**WO 2022/090389 (05.05.2022 Gazette 2022/18)**

(54) **METHOXYPROPANOLS SEPARATION COMBINING MEMBRANE SEPARATION AND DISTILLATION**

METHOXYPROPANOLTRENNUNG DURCH KOMBINATION VON MEMBRANTRENNUNG UND DESTILLATION

SÉPARATION DE MÉTHOXYPROPANOL COMBINANT LA SÉPARATION ET LA DISTILLATION DE MEMBRANE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.10.2020 EP 20204567**

(43) Date of publication of application:
**06.09.2023 Bulletin 2023/36**

(73) Proprietors:
• **BASF SE**
**67056 Ludwigshafen (DE)**
• **Dow Global Technologies LLC**
**Midland, MI 48674 (US)**

(72) Inventors:
• **RIEDEL, Dominic**
**67056 Ludwigshafen am Rhein (DE)**
• **SAN PIO BORDEJE, Maria Angel**
**67056 Ludwigshafen am Rhein (DE)**
• **SEGERS, Dylan**
**2040 Antwerpen (BE)**
• **TELES, Joaquim Henrique**
**67056 Ludwigshafen am Rhein (DE)**

• **KAPPERT, Emiel Jan**
**67056 Ludwigshafen am Rhein (DE)**
• **DE OLIVEIRA, Ana Luiza**
**91054 Erlangen (DE)**
• **WEIDENBACH, Meinolf**
**21683 Stade (DE)**
• **VAN NEER, Franciscus Johannes Robertus**
**4542 NM Hoek (NL)**

(74) Representative: **Altmann, Andreas**
**Altmann Stößel Dick Patentanwälte PartG mbB**
**Isartorplatz 1**
**80331 München (DE)**

(56) References cited:
**EP-A1- 1 375 462**    **US-A1- 2004 000 473**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 41/36, C07C 43/13;
C07C 41/42, C07C 43/13**

**Description**

A first aspect of the invention relates to a process for separating 1-methoxypropan-2-ol from an aqueous stream comprising 1-methoxypropan-2-ol and 2-methoxypropan-1-ol, wherein the process comprises providing a stream S0 comprising 1-methoxypropan-2-ol and 2-methoxypropan-1-ol, water, and having a molar ratio of 1-methoxypropan-2-ol : 2-methoxypropan-1-ol in the range of from 1 : 5 to 5 : 1; wherein the final stream S5 comprises ≥ 95 weight-% 1-methoxypropan-2-olbased on the total weight of S5.

Propylene oxide (PO) is one of the most important chemical intermediates in industry. It represents the starting compound for a broad spectrum of products, such as foams, solvents or deicing agents. Traditionally, PO is produced via the chlorohydrin process, which is still in use today, as well as the oxirane method. The development of catalysts based on zeolitic materials having a framework structure comprising Si, O, and Ti, such as titanium silicalite-1, together with the improved availability of large quantities of hydrogen peroxide enabled the large-scale implementation of the co-product-free HPPO technology. This new process enables PO to be produced with excellent yields and selectivities.

The HPPO process produces propylene oxide from propylene and hydrogen peroxide in aqueous organic solvents with zeolitic materials having a framework structure comprising Si, O, and Ti as catalysts, in one constellation, methanol is used as the solvent, typically in combination with a zeolitic material having a framework structure comprising Si, O, and Ti of framework type MFI (titanium silicalite-1, TS-1) as catalyst. As side products in the HPPO process, methoxypropanols (MOPs) are formed by reaction of methanol (MeOH) and PO. MOPs are formed as a mixture of the isomers 1-methoxypropan-2-ol and 2-methoxypropan-1-ol. Therefore, 1-methoxypropan-2-ol is an interesting compound, which can be used, for example, as a solvent. 2-Methoxypropan-1-ol on the other hand is teratogenic and has to be removed.

In an HPPO plant, MeOH, after having been used as solvent in the formation of PO, is normally separated from undesired side components and recovered. The MeOH containing stream, as mentioned above, comprises several side products such as the 1-methoxypropan-2-ol, which are of interest for further use. However, due to the similarities between the side products, the isolation of pure 1-methoxypropan-2-ol is complicated. First, the separation of both isomers is a difficult task, especially when considering the low concentration of water that is required for the final product. Second, it is quite complicated to get rid of other side products, for example, propylene glycol dimethyl ether.

US 5,723,024 A describes a method for recovering 2-methyl-1-propanol from a mixture consisting of 2-methyl-1-propanol and 1-butanol. EP 0 425 893 A discloses a separation method for the two MOP isomers. DE 10233388 A1 describes MOP separation in general, but not specifically the separation of both MOP isomers. US 2004/0000473 A1 refers to the separation of the two MOP isomers from aqueous solution by usage of dewatering agents, without separation of both isomers. EP 1 375 462 A1 refers to a process of separating 1-methoxy-2-propanol and 2-methoxy-1-propanol from aqueous compositions, comprising dewatering of the aqueous composition comprising 1-methoxy- 2-propanol and 2-methoxy-1-propanol to a concentration of 1-methoxy-2-propanol and 2-methoxy-1-propanol of at least 90 percent by weight in total and isolation of 1-methoxy-2-propanol, 2-methoxy-1-propanol or mixtures thereof by means of distillation. The dewatering can be done by azeotropic distillation using an additive or by extractive distillation. The aqueous composition can be concentrated prior to the dewatering step by means of a pre-distillation to contain at least 10 percent by weight in total of 1-methoxy-2-propanol and 2-methoxy-1-propanol. The distillate obtained by such a distillation preferably contains the MOP isomers in an amount of 10 to 50 percent by weight. According to EP 1 375 462 A1, any suitable distillation column can be used for the pre-distillation step. The distillation column used for the pre-distillation can, according to EP 1 375 462 A1, be run at any suitable pressure; however, a pressure in the range of 0.5 to 5 bar is indicated as preferred embodiment, whereas atmospheric pressure is indicated as most preferred. Membrane technology is mentioned regarding separation methods for example for separation of propylene glycol monomethyl ether and water, but only general concepts are given (CN 103342631 A, CN 103992214 A).

In view of the plurality of side products, which can be present in a stream comprising the two MOP isomers, there is still a need to separate the 1-methoxypropan-2-ol in high purity. Especially, it had been found that removal of specific impurities, such as propylene glycol dimethyl ether, cannot, or at least not efficiently, be achieved by the methods known in the art.

It was therefore an object of the present invention to provide a process for 1-methoxypropan-2-ol separation, which is effective and allows to achieve 1-methoxypropan-2-ol in high purity with only traces of water, as well as only traces of impurities.

In a first aspect, the invention thus relates to a process for separating 1-methoxypropan-2-ol from an aqueous stream comprising 1-methoxypropan-2-ol and 2-methoxypropan-1-ol, wherein the process comprises:

(a) Providing a stream S0 comprising 1-methoxypropan-2-ol, 2-methoxypropan-1-ol and water, and having a molar ratio of 1-methoxypropan-2-ol : 2-methoxypropan-1-ol in the range of from 1 : 5 to 5 : 1;
(b) separating 1-methoxypropan-2-ol and 2-methoxypropan-1-ol from the stream S0 provided in (a) by distillation comprising subjecting the stream S0 provided in (a) to distillation conditions in a distillation unit comprising a distillation column B, obtaining a (top) stream S1 comprising 1-methoxypropan-2-ol, 2-methoxypropan-1-ol and water, which is enriched in 1-methoxypropan-2-ol and 2-methoxypropan-1-ol compared to the stream S0 and a bottoms stream S1a comprising water and being depleted of 1-methoxypropan-2-ol and 2-methoxypropan-1-ol compared to S0; wherein the distillation column B is operated at a pressure of $\geq 2$ bar;

(c.1) separation of the stream S1 obtained in (b) with at least one membrane unit M comprising at least one membrane module, obtaining a stream S2 which is depleted of water and further enriched in 1-methoxypropan-2-ol and 2-methoxypropan-1-ol compared to the stream S1, and a stream S2a comprising water;

(c.2) subjecting the stream S2 obtained in (c.1) to distillation conditions in a distillation unit comprising a distillation column C, obtaining a stream S3, which is depleted of water and further enriched in 1-methoxypropan-2-ol and 2-methoxypropan-1-ol compared to the stream S2, and a stream S3a comprising water;

(d) separating 1-methoxypropan-2-ol from the stream S3 obtained in (c.2) by distillation, comprising subjecting the stream S2 obtained in (c.2) to distillation conditions in a distillation unit comprising a distillation column D, obtaining a stream S5 comprising $\geq 95$ weight-% 1-methoxypropan-2-ol and $\leq 0.5$ weight-% of 2-methoxypropan-1-ol, based on the total weight of stream S5, and a stream S4 comprising $\geq 95$ weight-% 2-methoxypropan-1-ol based on the total weight of stream S4;

(e) optionally recirculating at least a part of the stream S3a to (c.1).

Preferably, stream S0 comprises $\geq 0.001$ weight-% propylene glycol dimethyl ether (1,2-dimethoxypropane) based on the total weight of S0; and stream S5 preferably comprises $\leq 0.01$ weight-% of propylene glycol dimethyl ether based on the total weight of S5.

Surprisingly, it was found that if the distillation column B is operated at a pressure of $\geq 2$ bar, it is possible to separate the propylene glycol dimethyl ether very efficiently, without need for further separation stages. It was found that operating column B at 1 bar (about atmospheric pressure) resulted in more water going over the top of column B with stream S1, which included the isomers 1-methoxypropan-2-ol and 2-methoxypropan-1-ol to the membrane unit M and optionally also to column C. Further, more impurities were transferred together with the water/1-methoxylpronan-2-ol, 2-methoxypropan-1-ol azeotrope to membrane unit M; mostly propylene glycol dimethyl ether. Already in stream S1, the content of propylene glycol dimethyl was $9.18 \times 10^{-3}$ weight-% based on the weight of S1 when column B was operated at 1 bar, compared to operating distillation column B at a pressure of $\geq 2$ bar (10 bar), where the content of propylene glycol dimethyl was only $1.56 \times 10^{-3}$ weight-% based on the weight of S1, i.e. the impurity content in stream S1 was factor 5.7 more when column B was operated at 1 bar compared to operation at 10 bar. Consequently, also the impurity content in the final stream S5 was higher when column B was operated at 1 bar, resulting in a slightly lower purity of the obtained 1-methoxypropanol-2, and, simultaneously, in a propylene glycol dimethyl ether content in S5 of more than 0.01 weight-% based on the total weight of S5.

Preferably, (a), (b), (c.1), (c.2) and (d) and optionally also the recycling (e) are operated in batch mode or in continuous mode, more preferred a (a), (b), (c.1), (c.2) and (d) and optionally also the recycling (e) are operated in continuous mode.

The process is preferably conducted in a continuous mode and is energy efficient. Thus, in a preferred embodiment of the process for separating 1-methoxypropan-2-ol from an aqueous stream comprising 1-methoxypropan-2-ol and 2-methoxypropan-1-ol, the thermal energy of stream S1 is partly transferred to a heat transfer medium stream HTMS1 after (b) and before step (c), preferably in a heat exchanger H, obtaining a heat transfer medium stream HTMS1a having an increased thermal energy content compared to HTMS1;

- wherein HTMS1a is used to provide thermal energy to:

- the membrane unit of (c.1), preferably to a heat exchanger of the membrane unit of (c.1);
  and/or

- the distillation unit of step (c.2), preferably to a heat exchanger unit connected to the distillation column C of the distillation unit of (c.2),
  and/or

- the distillation unit of (d), preferably to a heat exchanger unit connected to the distillation column of the distillation unit of (d).

According to a further preferred embodiment of the process for separating 1-methoxypropan-2-ol from an aqueous stream comprising 1-methoxypropan-2-ol and 2-methoxypropan-1-ol, HTMS1a is used to provide thermal energy to:

- the membrane unit of (c.1), preferably to a heat exchanger of the membrane unit of (c.1); and

- the distillation unit of step (c.2), preferably to a heat exchanger unit connected to the distillation column C of the distillation unit of (c.2), and/or

- the distillation unit of (d), preferably to a heat exchanger unit connected to the distillation column of the distillation unit of (d).

According to a further preferred embodiment of the process for separating 1-methoxypropan-2-ol from an aqueous stream comprising 1-methoxypropan-2-ol and 2-methoxypropan-1-ol, HTMS1a is used to provide thermal energy to:

- the membrane unit of (c.1), preferably to a heat exchanger of the membrane unit of (c.1); and

- the distillation unit of step (c.2), preferably to a heat exchanger unit connected to the distillation column C of the distillation unit of (c.2), and

- the distillation unit of (d), preferably to a heat exchanger unit connected to the distillation column of the distillation unit of (d).

The membrane unit of (c.1) comprises at least one membrane, preferably at least one membrane and a heat exchanger, more preferred at least one membrane, a heat exchanger, a pump and a vacuum system.

According to a further preferred embodiment of the process for separating 1-methoxypropan-2-ol from an aqueous stream comprising 1-methoxypropan-2-ol and 2-methoxypropan-1-ol, the thermal energy provided by HTMS1a provides at least 90 % of the energy demand of the membrane unit of (c.1), and/or of the distillation unit of (c.2),and/or of the distillation unit of (d), preferably the thermal energy provided by HTMS1a provides at least 95 % of the energy demand of the membrane unit of (c.1), and/or of the distillation unit of (c.2),and/or of the distillation unit of (d); more preferably the thermal energy provided by HTMS1a provides at least 98 %, more preferably at least 99 %, more preferably 100 %, of the energy demand of the membrane unit of (c.1), and/or of the distillation unit of (c.2),and/or of the distillation unit of (d).

According to a further preferred embodiment of the process for separating 1-methoxypropan-2-ol from an aqueous stream comprising 1-methoxypropan-2-ol and 2-methoxypropan-1-ol, the thermal energy provided by HTMS1a provides at least 90 % of the energy demand of the membrane unit of (c.1), and of the distillation unit of (c.2),and of the distillation unit of (d), preferably the thermal energy provided by HTMS1a provides at least 95 % of the energy demand of the membrane unit of (c.1), and of the distillation unit of (c.2),and of the distillation unit of (d); more preferably the thermal energy provided by HTMS1a provides at least 98 % more preferably at least 99 %, more preferably 100 %, of the energy demand of the membrane unit of (c.1), and of the distillation unit of (c.2), and of the distillation unit of (d).

According to a further preferred embodiment of the process for separating 1-methoxypropan-2-ol from an aqueous stream comprising 1-methoxypropan-2-ol and 2-methoxypropan-1-ol, in the range of from 40 to 95% of the thermal energy of stream S1 is transferred to the heat transfer medium stream HTMS1 obtaining a heat transfer medium stream HTMS1a having an increased thermal energy content compared to HTMS1.

Preferably, in the range of from 50 to 90 %, more preferred in the range of from 55 to 85 %, more preferred in the range of from 60 to 80 %, of the thermal energy of stream S1 is transferred to the heat transfer medium stream HTMS1. A transfer of thermal energy of, for example, 70 % means that, for example, stream S1 has a temperature of 177 °C before thermal energy transfer and of 120 °C after thermal energy transfer. This corresponds to an energy content of 9.18 MW, wherein 1.34 MW are transferred via HTMS1/HTHMS1a to the membrane unit of (c.1), and 197.14kW to the distillation unit of (c.2), and 1121kW to the distillation unit of (d).

The heat transfer medium used for HTMS1/HTMS1a and for HTMS2/HTMS2a is preferably steam ($H_2O_{gaseous}$).

It was surprisingly found that if column B is operated at a pressure $\geq 2$ bar, the involved units can be energetically coupled in a very efficient manner. At lower pressure, it is impossible to energetically couple the units, causing the energy consumption to increase drastically.

Stream S0

According to step (a), a stream S0 is provided, which comprises 1-methoxypropan-2-ol, 2-methoxypropan-1-ol and water, and which has a molar ratio of 1-methoxypropan-2-ol : 2-methoxypropan-1-ol in the range of from 1 : 5 to 5 : 1. According to a preferred embodiment, stream S0 is a stream obtained from a HPPO process, more preferred from an HPPO process, wherein propylene oxide is prepared from propylene and hydrogen peroxide in aqueous methanol as solvent, preferably using a TS-1 catalyst, more preferred using a TS-1 fixed bed catalyst in a reaction zone. MOPs are formed as side products in the process by reaction of MeOH and PO and are removed from the reaction zone with an initial MeOH containing product stream. From said initial product stream, MeOH is recovered, optionally using one or more further separation steps. According to one embodiment, an intermediate stream comprising MeOH, water and other components including the two MOP isomers is obtained. MeOH is then separated from water and the other components via distillation, wherein stream S0 is obtained, which comprises the two MOP isomers, water, and several other side components such as propylene glycol dimethyl ether (1 ,2-dimethoxypropane). In a preferred embodiment of the process for separating 1-methoxypropan-2-ol from an aqueous stream comprising 1-methoxypropan-2-ol and 2-methoxypropan-1-ol, stream S0 provided in a) comprises water in an amount in the range of from 50 to 90 weight-%, preferably in the range of from 55 to 85 weight-% and a mixture of 1-methoxypropan-2-ol and 2-methoxypropan-1-ol in an amount in the range of from 8 to 50 weight-%, preferably in the range of from 13 to 45 weight-%, each based on the total weight of stream S0, the remaining amount up to 100 weight-% being other components (impurities and solvent (MeOH)). Preferably, stream S0 provided in a) comprises 1-methoxypropan-2-ol and 2-methoxypropan-1-ol in a molar ratio in the range of from 1 : 4 to 4 : 1; more preferred in the range of from 1 : 3 to 3 : 1, more preferred in the range of from 1 : 2 to 2 : 1, more preferred in the range of from 0.75 : 1 to 1 : 0.75. Preferably, stream S0 provided in a) comprises propylene glycol dimethyl ether in an amount of $\geq$ 0.001 weight-%, more preferred in the range of from 0.001 to 0.1 weight-%, more preferred in the range of from 0.003 to 0.01 weight-%, more preferred in the range of from 0.004 to 0.01 weight-%, more preferred in the range of from 0.005 to 0.008 weight-%, based on the total weight of S0. Propylene glycol dimethyl ether (1,2-dimethoxypropane) is an impurity contained in S0. Preferably, stream S0 comprises besides propylene glycol dimethyl ether in the range of from 0.0001 to 3 weight-%, more preferably in the range of from 0.001 to 2.5 weight-%, more preferred in the range of from 0.01 to 1.5 weight-%, each based on the total weight of S0, of one or more components selected from the group consisting of 1,1-dimethoxypropane, 1,2-propanediol (MPG), 1-butanol, 2,4-dimethyl-1,3-dioxolane, 2,6-dimethyl-4-heptanol, 2-butenal,2-ethyl-4-methyl-1,3-dioxolane, 2-hexanone, 2-methylcyclohexanol, 2-methylpentanal, 2-propen-1-ol, 4-methyl-1,3- dioxolane, acetaldehyde, 2-propanone, dimethoxymethane, dipropylene glycol, ethanol, hydroxyacetone , 2-propanol, methanol, acetic acid methyl ester, formic acid methyl ester, propylene oxide, tripropylene glycol, and dipropylene glycol monomethyl ether (DPGME). These components are here understood as solvent (MeOH) and further impurities.

Column B

According to step (b), 1-methoxypropan-2-ol and 2-methoxypropan-1-ol are separated from the stream S0 provided in (a) by distillation comprising subjecting the stream S0 provided in (a) to distillation conditions in a distillation unit comprising a distillation column B. Preferably, the distillation column B comprised in the distillation unit according to b) has between 5 and 100 theoretical stages, preferably between 8 and 60 theoretical stages, more preferred between 10 and 40 theoretical stages, more preferred between 15 and 30 theoretical stages. Preferably, the distillation column B comprised in the distillation unit according to b) is operated at a pressure in the range of from 2 to 30 bar, more preferred in the range of from 2.5 to 20 bar, more preferred in the range of from 3 to 15 bar, more preferred in the range of from 3.5 to 14 bar, more preferred in the range of from 4 to 12 bar, more preferred in the range of from 5 to 11 bar. Regarding specific temperatures to be used at the top or at the bottom of distillation column B, no specific restrictions exists as long as a stream S1 and a stream S1a according to (b) are obtained. Preferably, the distillation column B comprised in the distillation unit according to b) is operated at a temperature at the top of the distillation column B in the range of from 140 to 250 °C, more preferred in the range of from 150 to 220 °C, more preferred in the range of from 160 to 200 °C, more preferred in the range of from 170 to 190 °C. Preferably, the distillation column B comprised in the distillation unit according to b) is operated at a temperature at the bottom in the range of from 140 to 250 °C, more preferred in the range of from 150 to 220 °C, more preferred in the range of from 160 to 200 °C, more preferred in the range of from 170 to 190 °C. Regarding specific reflux conditions to be used in the distillation column B, no specific restrictions exists as long as a stream S1 and a stream S1a according to (b) are obtained. Preferably, the distillation column B comprised in the distillation unit according to b) is operated with a reflux ratio in the range of from 1 to 10 g/g, more preferred in the range of from 2 to 9 g/g, more preferred in the range of from 3 to 8 g/g, more preferred in the range of from 4 to 6 g/g. According to a preferred embodiment of the process for separating 1-methoxypropan-2-ol from an aqueous stream comprising 1-methoxypropan-2-ol and 2-methoxypropan-1-ol, the distillation column B comprised in the distillation unit according to b) has an energy demand in the range of from 10 to 30 MW; wherein preferably the reboiler of distillation column B has an energy demand in the range of from 5 to 15 MW and the condenser of distillation column B has an energy demand the range of from 5 to 15 MW.

Stream S1

In step (b), a (top) stream S1 comprising 1-methoxypropan-2-ol, 2-methoxypropan-1-ol and water, which is enriched in 1-methoxypropan-2-ol and 2-methoxypropan-1-ol compared to the stream S0 is obtained. Preferably, $\geq$ 95 weight-% of stream S1, which leaves distillation column B over the top, consist of water, 1-methoxypropan-2-ol and 2-methoxypropan-1-ol; wherein more preferred stream S1 comprises water in an amount in the range of from 40 to 80 weight-%, preferably in the range of from 50 to 70 weight-%, more preferred in the range of from 55 to 65 weight-%; and a mixture of 1-methoxypropan-2-ol and 2-methoxypropan-1-ol in an amount in the range of from 20 to 60 weight-%, preferably in the range of from 30 to 50 weight-%, more preferred in the range of from 35 to 45 weight-%, each based on the total weight of stream S1, wherein preferably stream S1 comprises 1-methoxypropan-2-ol and 2-methoxypropan-1-ol in a molar ratio in the range of from 1 : 4 to 4 : 1; preferably in the range of from 1 : 3 to 3 : 1, more preferred in the range of from 1 : 2 to 2 : 1, more preferred in the range of from 0.75 : 1 to 1 : 0.75. Preferably, stream S1, which leaves distillation column B over the top, contains less than 0.05 weight-%, more preferred less than 0.005 weight-%, more preferred less than 0.004 weight-%, more preferred less than 0.002 weight-%, of propylene glycol dimethyl ether, based on the total weight of S1. The wording "less than" means from 0 to less than the respective value. Preferably, stream S1 after leaving column B and before entering the membrane unit M is flashed [to reduce the heat demand in column C]. If stream S1 after leaving column B and before entering the membrane unit M is flashed, the membrane unit operates in "vapor permeation mode".

Stream S1a

In step (b), a bottoms stream S1a comprising water and being depleted of 1-methoxypropan-2-ol and 2-methoxypropan-1-ol compared to S0 is obtained. Preferably, stream S1a comprises $\geq$ 95 weight-% of water, and preferably less than 1 weight-% of stream S1a consist of 1-methoxypropan-2-ol and 2-methoxypropan-1-ol, each based on the total weight of stream S1a, more preferred S1a comprises $\geq$ 98 weight-% of water, and less than 1 weight-% of stream S1a consist of 1-methoxypropan-2-ol and 2-methoxypropan-1-ol; more preferred S1a comprises $\geq$ 99 weight-% of water, and less than 1 weight-% of stream S1a consist of 1-methoxypropan-2-ol and 2-methoxypropan-1-ol. Preferably, bottoms stream S1a is afterwards send to a subsequent water treatment or recycled into one or more stages of the process or recycled into one or more stages of an upstream HPPO process, from which stream S0 derives, more preferred S1a is sent to water treatment or recycled into one or more stages of an upstream HPPO process, from which stream S0 derives.

Membrane unit M

In step (c.1), the stream S1 obtained in (b) is separated with at least one membrane unit M comprising at least one membrane module. Preferably, the membrane unit M according to (c.1) comprises one or more membrane modules, each membrane module comprising at least one membrane, and optionally one or more further component selected from the group consisting of heat exchanger, pump compressor and condenser. Preferably, there are means present at the permeate side of the at least one membrane which are able to provide a vacuum. In some preferred embodiments, the means is a vacuum system, which comprises from the above identified components at least a condenser and a pump. According to a preferred embodiment of the process for separating 1-methoxypropan-2-ol from an aqueous stream comprising 1-methoxypropan-2-ol and 2-methoxypropan-1-ol, the membrane(s) comprised in the one or more membrane modules, which are comprised in the membrane unit M according to (c.1) comprise(s) a material selected from the group consisting of polymers; inorganic materials, preferably zeolites; carbon materials; hybrid inorganic-organic materials, preferably hybrid silica materials; and composite materials; wherein the material of each membrane may be the same or different. Preferably, the material comprised in the membrane is selected so that water -compared to all other components- is preferably passed.

Preferably, the membrane(s) has/ have a permselectivity for water over 1-methoxypropan-2-ol and over 2-methoxypropan-1-ol greater than 1. In principle, there are two permselectivities: water/1-methoxypropan-2-ol and water/2-methoxypropan-1-ol, however, these two permselectivities deviate from each other by less than 5%. The permselectivity ($\alpha$) of a component a over a component b ($\alpha_{a/b}$) is defined as the ratio of the permeances of these components: $\alpha_{a/b} = F_a/F_b$. The permeance (F) of a component is defined as the flux normalized to the difference in partial vapor pressure over the membrane. For example, for a component a the permeance $F_a$ is defined as follows:

$$F_a = \text{Flux}_a \, / \, (p_{a,ret} - p_{a,perm}),$$

wherein $p_{a,ret}$ is the vapor pressure of a component a at the retentate side.

The average flux through the membrane(s) is in the range of 1 to 100 kg/m$^2$/h, preferably in the range of 3 to 30 kg/m$^2$/h, more preferred in the range of 5-15 kg/m$^2$/h.

Preferably, the membrane unit M according to (c.1) is operated in pervaporation mode or vapor permeation mode, preferably in pervaporation mode. Preferably, the one or more membrane module(s) of the membrane unit M according to (c.1) are operated under pervaporation conditions and are arranged in loops that preferably comprise, more preferred consist of, at least a crossflow pump, a heat exchanger, and one or more membrane modules; wherein the number of loops is preferably in the range of from 1 to 10 loops, preferably in the range of from 1 to 4 loops, more preferred in the range of from 2 to 3 loops. Preferably, the one or more membrane module(s) of the membrane unit M according to (c.1) are operated under pervaporation conditions and are connected in series with interstage heat exchangers. Preferably, the one or more membrane modules of the membrane unit M according to (c.1) operate at a temperature in the range of from 70 to 160 °C, more preferred in the range of from 90 to 140 °C, more preferred in the range of from 110 to 130 °C. Preferably, the permeate sides of one or more membrane modules of the membrane unit M according to (c.1) are operated at a vacuum pressure in the range of from 10 mbar(a) to atmospheric pressure, more preferred in the range of from 20 to 200 mbar(a), more preferred in the range of from 30 to 80 mbar(a), and/or, preferably and, the retentate sides of one or more membrane modules of the membrane unit M according to (c.1) are operated at a pressure in the range of from 0.5 to 10 bar, preferably in the range of from 1 to 5 bar, more preferred in the range of from 1.5 to 2.5 bar, wherein pressure at the retentate side is higher than the pressure at the permeate side.

Stream S2

In step (c.1) the stream S1 obtained in (b) is separated with at least one membrane unit M comprising at least one membrane module, obtaining a stream S2 which is depleted of water and further enriched in 1-methoxypropan-2-ol and 2-methoxypropan-1-ol compared to the stream S1. Preferably, the stream S2 is obtained from membrane unit M, more preferred from the retentate side of the membrane unit M, and comprises water, 1-methoxypropan-2-ol and 2-methoxypropan-1-ol. Preferably, in in the range of from 55 to 98 weight-% of S2 consists of 1-methoxy-2-propanol and 2-methoxy-1-propanol based on the total weight of S2, wherein more preferred stream S2 comprises 1-methoxypropan-2-ol and 2-methoxypropan-1-ol in a molar ratio in the range of from 1 : 4 to 4 : 1; more preferred in the range of from 1 : 3 to 3 : 1, more preferred in the range of from 1 : 2 to 2 : 1, more preferred in the range of from 0.75 : 1 to 1 : 0.75. Preferably. the stream S2 comprises water in the range of from 0.1 to 45 weight-%, more preferred in the range of from 0.5 to 10 weight-%, more preferred in the range of from 3 to 5 weight-%, more preferred in the range of from 3.5 to 4.5 weight-%, based on the total weight of S2. Preferably, stream S2 after leaving the membrane unit M and before entering column C is flashed [to reduce the heat demand in column C].

Stream S2a

In step (c.1), the stream S1 obtained in (b) is separated with at least one membrane unit M comprising at least one membrane module, obtaining a stream S2a comprising water. Preferably, the stream S2a is obtained from membrane unit M, wherein 90 weight-%, more preferred ≥ 95 weight-%, more preferred ≥97 weight-%, ≥ 98 weight-%, more preferred ≥ 99 weight-% of S2a consisted of water based on the total weight of S2a.

Column C

In step (c.2), the stream S2 obtained in (c.1) is subjected to distillation conditions in a distillation unit comprising a distillation column C, obtaining a stream S3, which is depleted of water and further enriched in 1-methoxypropan-2-ol and 2-methoxypropan-1-ol compared to the stream S2, and a stream S3a comprising water. Preferably, the distillation column C comprised in the distillation unit according to (c.2) has between 2 and 50 theoretical stages, more preferred between 5 and 25 theoretical stages, more preferred between 10 and 20 theoretical stages. Preferably, the distillation column C comprised in the distillation unit according to (c.2) is operated at a pressure in the range of from 0.5 to 10 bar, more preferred in the range of from 1 to 5 bar, more preferred in the range of from 1.5 to 2.5 bar. Preferably, the distillation column C comprised in the distillation unit according to (c.2) is operated at a bottoms temperature in the range of from 120 to 180 °C, more preferred in the range of from 130 to 170 °C, more preferred in the range of from 140 to 160 °C. Preferably, the distillation column C comprised in the distillation unit according to (c.2) is operated at a temperature at the top of the column in the range of from 70 to 110 °C, more preferred in the range of from 80 to 100 °C, more preferred in the range of from 85 to 95 °C. Preferably, the distillation column C comprised in the distillation unit according to (c.2) is operated with a reflux ratio in the range of from 10 to 40 g/g, more preferred in the range of from 12 to 30 g/g, more preferred in the range of from 15 to 25 g/g.

Stream S3

In step (c.2), a stream S3 is obtained, which is depleted of water and further enriched in 1-methoxypropan-2-ol and 2-methoxypropan-1-ol compared to the stream S2. Preferably, stream S3 comprises water, 1-methoxypropan-2-ol and 2-methoxypropan-1-ol, wherein S3 more preferred comprises ≤ 1 weight-%, more preferred ≤ 0.1 weight-%, water, based on the total weight of S3. Preferably, stream S3 comprises ≥95 weight-%, more preferred ≥96 weight-%, more preferred ≥ 97 weight-%, more preferred ≥98 weight-%, more preferred ≥99 weight-%, of 1-methoxypropan-2-ol and 2-methoxypropan-1-ol, based on the total weight of S3; and/or, preferably and, wherein S3 has a water content of at most 100 ppm, based on the total weight of S3. Preferably, stream S3 comprises 1-methoxypropan-2-ol and 2-methoxypropan-1-ol in a molar ratio in the range of from 1 : 4 to 4 : 1; more preferred in the range of from 1 : 3 to 3 : 1, more preferred in the range of from 1 : 2 to 2 : 1, more preferred in the range of from 0.75 : 1 to 1 : 0.75

Stream 3a

In step (c.2), a stream S3a comprising water is obtained. Preferably, in step (e) at least 50 weight-% of S3a, more preferred at least 60 weight-% of S3a, more preferred at least 70 weight-% of S3a, more preferred at least 80 weight-% of S3a, more preferred at least 90 weight-% of S3a, more preferred at least 95 weight-% of S3a, more preferred at least 99 weight-% of S3a, more preferred at least 99.5 weight-% of S3a, more preferred at least 100 weight-% of S3a, are recycled to the membrane unit M of (c.1). Preferably, stream S3a comprises water, 1-methoxypropan-2-ol and 2-methoxypropan-1-ol, wherein more preferred S3a comprises less than 10 weight-% of water, based on the total weight of S3a, and/or more than 80 weight-% of S3a are 1-methoxypropan-2-ol and 2-methoxypropan-1-ol. Preferably, stream S3a comprises ≥ 70 weight-%, more preferred ≥ 75 weight-%, more preferred ≥ 80 weight-%, 2-methoxypropan-1-ol, based on the total weight of S3a; and/or wherein stream S3a comprises ≤ 20 weight-%, preferably ≤ 15 weight-%, more preferred ≤ 10 weight-%, 1-methoxypropan-2-ol, based on the total weight of S3a.

Column D

In step (d), 1-methoxypropan-2-ol is separated from the stream S3 obtained in (c.2) by distillation, comprising subjecting the stream S2 obtained in (c.2) to distillation conditions in a distillation unit comprising a distillation column D. Preferably, the distillation column D comprised in the distillation unit according to (d) has between 20 and 100 theoretical stages, more preferred between 30 and 80 theoretical stages, more preferred between 40 and 60 theoretical stages, more preferred between 45 and 52 theoretical stages. Preferably, the distillation column D comprised in the distillation unit according to (d) is operated at a pressure in the range of from 0.5 to 10 bar, more preferred in the range of from 1 to 5 bar, more preferred in the range of from 2 to 4 bar. Preferably, the distillation column D comprised in the distillation unit according to (d) is operated at a bottoms temperature in the range of from 140 to 200 °C, more preferred in the range of from 150 to 190 °C, more preferred in the range of from 160 to 180 °C. Preferably, the distillation column D comprised in the distillation unit according to (d) is operated at a temperature at the top of the column in the range of from 120 to 200 °C, more preferred in the range of from 130 to 180 °C, more preferred in the range of from 140 to 165 °C. Preferably, the distillation column D comprised in the distillation unit according to (d) is operated with a reflux ratio in the range of from 5 to 30 g/g, more preferred in the range of from 8 to 20 g/g, more preferred in the range of from 10 to 15 g/g. Preferably, the distillation column D comprised in the distillation unit according to (d) has an energy demand in the range of from 1 to 5 MW; wherein more preferred the reboiler of distillation column D has an energy demand in the range of from 1 to 1.5 MW and the condenser of distillation column D has an energy demand the range of from 1 to 1.5 MW. For example, having 48 theoretical stages in distillation column D would result in an energy demand of 1.245 MW in the reboiler of distillation column D and 1.23 MW in the condenser of distillation column D. In case of decreasing the theoretical stages from 48 to 40, the heat duty would increase from 1.245 MW to 12 MW in the reboiler, i.e. the energy demand would increase by about factor 10.

Stream S4

In step (d), a stream S4 comprising ≥ 95 weight-% 2-methoxypropan-1-ol based on the total weight of stream S4 is obtained. Preferably, the stream S4 is removed as bottoms stream from distillation column D, wherein S4 comprises ≥ 95 weight-% 2-methoxypropan-1-ol and ≤ 0.5 weight- % 1-methoxypropan-2-ol, preferably ≥ 96 weight-% 2-methoxypropan-1-ol and ≤ 0.1 weight- % 1-methoxypropan-2-ol, more preferred ≥ 98 weight-% 2-methoxypropan-1-ol and ≤ 0.001 weight- % 1-methoxypropan-2-ol, more preferred ≥ 99 weight-% 2-methoxypropan-1-ol and ≤ 0.0001 weight- % 1-methoxypropan-2-ol, each based on the total weight of stream S4.
1-Methoxypropan-2-ol is a valuable product. Thus, its loss should be kept minimal. Based on the process of the present invention, the following specifications in stream S4 can be met: 0.05 weight-% of 1-methoxypropan-2-ol in S4 based on the total weight of S4.

Stream S5

In step (d), also a stream S5 comprising ≥ 95 weight-% 1-methoxypropan-2-ol and ≤ 0.5 weight-% of 2-methoxypropan-1-ol, based on the total weight of stream S5 is obtained. Preferably, the stream S5, which is removed as top stream from distillation column D, comprises ≥ 98 weight-% 1-methoxypropan-2-ol and ≤ 0.15 weight- % of 2-methoxypropan-1-ol, more preferred ≥ 99 weight-% 1-methoxypropan-2-ol and ≤ 0.1 weight- % of 2-methoxypropan-1-ol, each based on the total weight of stream S5. Preferably, the stream S5 comprises ≤ 0.01 weight-% (in the range from 0 to 0.01 weight-%), more preferred ≤ 0.008 weight-% (in the range from 0 to 0.008 weight-%), more preferred ≤ 0.007 weight-% (in the range from 0 to 0.007 weight-%), more preferred ≤ 0.006 weight-% (in the range from 0 to 0.006 weight-%), of propylene glycol dimethyl ether based on the total weight of stream S5.

More preferred, stream S5 comprises the valuable product 1-methoxypropan-2-ol in a purity ≥ 95 weight-%, while the detrimental impurities 2-methoxypropan-1-ol and propylene glycol dimethyl ether are only present in amounts of ≤ 0.5 weight-% and < 0.01 weight-% respectively. Furthermore, stream S5 comprises less than 100 ppm, more preferred less than 50 ppm of water based on the total weight of stream S5.

2-methoxypropanol is a teratogenic component, thus, its concentration in the valuable 1-methoxypropanol stream has to be severely restricted (max 0.3 weight-%, based on the total weight of the respective stream). The following specification in stream S5 can be met: 0.03 weight-% 2-methoxypropanol in S5 based on the total weight of S5.

1-methoxypropan-2-ol

A further embodiment relates to 1-methoxypropan-2-ol obtained or obtainable from the process of the first aspect. Furthermore, an embodiment relates to a mixture of 1-methoxypropan-2-ol and 2-methoxypropan-1-ol, preferably obtained or obtainable from the process according to the process of the first aspect, which preferably comprises in the range of from 95 to 100 weight-% 1-methoxypropan-2-ol and ≤ 0.5 weight- % of 2-methoxypropan-1-ol, more preferred in the range of from 98 to 100 weight-% 1-methoxypropan-2-ol and ≤ 0.15 weight- % of 2-methoxypropan-1-ol, more preferred in the range of from 99 to 100 weight-% 1-methoxypropan-2-ol and ≤ 0.1 weight- % of 2-methoxypropan-1-ol, each based on the total weight of the mixture.

The present invention is further illustrated by the following set of embodiments and combinations of embodiments resulting from the dependencies and back-references as indicated. In particular, it is noted that in each instance where a range of embodiments is mentioned, for example in the context of a term such as "The ... of any one of embodiments 1 to 4", every embodiment in this range is meant to be explicitly disclosed for the skilled person, i.e. the wording of this term is to be understood by the skilled person as being synonymous to "The ... of any one of embodiments 1, 2, 3, and 4". Further, it is explicitly noted that the following set of embodiments is not the set of claims determining the extent of protection, but represents a suitably structured part of the description directed to general and preferred aspects of the present invention.

According to an embodiment (1), the present invention relates to a process for separating 1-methoxypropan-2-ol from an aqueous stream comprising 1-methoxypropan-2-ol and 2-methoxypropan-1-ol, wherein the process comprises:

(a) Providing a stream S0 comprising 1-methoxypropan-2-ol, 2-methoxypropan-1-ol and water, and having a molar ratio of 1-methoxypropan-2-ol : 2-methoxypropan-1-ol in the range of from 1 : 5 to 5 : 1:

(b) separating 1-methoxypropan-2-ol and 2-methoxypropan-1-ol from the stream S0 provided in (a) by distillation comprising subjecting the stream S0 provided in (a) to distillation conditions in a distillation unit comprising a distillation column B, obtaining a (top) stream S1 comprising 1-methoxypropan-2-ol, 2-methoxypropan-1-ol and water, which is enriched in 1-methoxypropan-2-ol and 2-methoxypropan-1-ol compared to the stream S0 and a bottoms stream S1a comprising water and being depleted of 1-methoxypropan-2-ol and 2-methoxypropan-1-ol compared to S0; wherein the distillation column B is operated at a pressure of ≥ 2 bar;

(c.1) separation of the stream S1 obtained in (b) with at least one membrane unit M comprising at least one membrane module, obtaining a stream S2 which is depleted of water and further enriched in 1-methoxypropan-2-ol and 2-methoxypropan-1-ol compared to the stream S1, and a stream S2a comprising water;

(c.2) subjecting the stream S2 obtained in (c.1) to distillation conditions in a distillation unit comprising a distillation column C, obtaining a stream S3, which is depleted of water and further enriched in 1-methoxypropan-2-ol and 2-methoxypropan-1-ol compared to the stream S2, and a stream S3a comprising water;

(d) separating 1-methoxypropan-2-ol from the stream S3 obtained in (c.2) by distillation, comprising subjecting the stream S2 obtained in (c.2) to distillation conditions in a distillation unit comprising a distillation column D, obtaining a stream S5 comprising ≥ 95 weight-% 1-methoxypropan-2-ol and ≤ 0.5 weight-% of 2-methoxypropan-1-ol, based on the total weight of stream S5, and a stream S4 comprising ≥ 95 weight-% 2-methoxypropan-1-ol based on the total weight of stream S4;

(e) optionally recirculating at least a part of the stream S3a to (c.1);

wherein preferably, stream S0 comprises≥ 0.001 weight-% propylene glycol dimethyl ether (1,2-dimethoxypropane) based on the total weight of S0; and stream S5 preferably comprises ≤ 0.01 weight-% of propylene glycol dimethyl ether based on the total weight of S5.

A preferred embodiment (2) concretizing embodiment (1) relates to said process for separating 1-methoxypropan-2-ol from an aqueous stream comprising 1-methoxypropan-2-ol and 2-methoxypropan-1-ol, wherein the thermal energy of stream S1 is partly transferred to a heat transfer medium stream HTMS1 after (b) and before step (c), preferably in a heat exchanger H, obtaining a heat transfer medium stream HTMS1a having an increased thermal energy content compared to HTMS1;

- wherein HTMS1a is used to provide thermal energy to:

- the membrane unit of (c.1), preferably to a heat exchanger of the membrane unit of (c.1);
  and/or

- the distillation unit of step (c.2), preferably to a heat exchanger unit connected to the distillation column C of the distillation unit of (c.2),
  and/or

- the distillation unit of (d), preferably to a heat exchanger unit connected to the distillation column of the distillation unit of (d).

A further preferred embodiment (3) concretizing embodiment (2) relates to said process for separating 1-methoxypropan-2-ol from an aqueous stream comprising 1-methoxypropan-2-ol and 2-methoxypropan-1-ol, wherein HTMS1a is used to provide thermal energy to:

- the membrane unit of (c.1), preferably to a heat exchanger of the membrane unit of (c.1); and

- the distillation unit of step (c.2), preferably to a heat exchanger unit connected to the distillation column C of the distillation unit of (c.2),
  and/or

- the distillation unit of (d), preferably to a heat exchanger unit connected to the distillation column of the distillation unit of (d).

A further preferred embodiment (4) concretizing embodiment (2) or (3) relates to said process for separating 1-methoxypropan-2-ol from an aqueous stream comprising 1-methoxypropan-2-ol and 2-methoxypropan-1-ol, wherein HTMS1a is used to provide thermal energy to:

- the membrane unit of (c.1), preferably to a heat exchanger of the membrane unit of (c.1);
  and

- the distillation unit of step (c.2), preferably to a heat exchanger unit connected to the distillation column C of the distillation unit of (c.2),
  and

- the distillation unit of (d), preferably to a heat exchanger unit connected to the distillation column of the distillation unit of (d).

A further preferred embodiment (5) concretizing any one of embodiments (2) to (4) relates to said process for separating 1-methoxypropan-2-ol from an aqueous stream comprising 1-methoxypropan-2-ol and 2-methoxypropan-1-ol, wherein the thermal energy provided by HTMS1a provides at least 90 % of the energy demand of the membrane unit of (c.1), and/or of the distillation unit of (c.2),and/or of the distillation unit of (d), preferably the thermal energy provided by HTMS1a provides at least 95 % of the energy demand of the membrane unit of (c.1), and/or of the distillation unit of (c.2),and/or of the distillation unit of (d); more preferably the thermal energy provided by HTMS1a provides at least 98

% more preferably at least 99 %, more preferably 100 %, of the energy demand of the membrane unit of (c.1), and/or of the distillation unit of (c.2),and/or of the distillation unit of (d).

A further preferred embodiment (6) concretizing any one of embodiments (2) to (5) relates to said process for separating 1-methoxypropan-2-ol from an aqueous stream comprising 1-methoxypropan-2-ol and 2-methoxypropan-1-ol, wherein the thermal energy provided by HTMS1a provides at least 90 % of the energy demand of the membrane unit of (c.1), and of the distillation unit of (c.2),and of the distillation unit of (d), preferably the thermal energy provided by HTMS1a provides at least 95 % of the energy demand of the membrane unit of (c.1), and of the distillation unit of (c.2),and of the distillation unit of (d); more preferably the thermal energy provided by HTMS1a provides at least 98 % more preferably at least 99 %, more preferably 100 %, of the energy demand of the membrane unit of (c.1), and of the distillation unit of (c.2),and of the distillation unit of (d).

A further preferred embodiment (7) concretizing any one of embodiments (2) to (6) relates to said process for separating 1-methoxypropan-2-ol from an aqueous stream comprising 1-methoxypropan-2-ol and 2-methoxypropan-1-ol, wherein in the range of from 40 to 95% of the thermal energy of stream S1 is transferred to the heat transfer medium stream HTMS1 obtaining a heat transfer medium stream HTMS1a having an increased thermal energy content compared to HTMS1.

A further preferred embodiment (8) concretizing any one of embodiments (1) to (7) relates to said process for separating 1-methoxypropan-2-ol from an aqueous stream comprising 1-methoxypropan-2-ol and 2-methoxypropan-1-ol, wherein stream S0 provided in a) comprises water in an amount in the range of from 50 to 90 weight-%, preferably in the range of from 55 to 85 weight-% and a mixture of 1-methoxypropan-2-ol and 2-methoxypropan-1-ol in an amount in the range of from 8 to 50 weight-%, preferably in the range of from 13 to 45 weight-%, each based on the total weight of stream S0, the remaining amount up to 100 weight-% being other components (impurities and solvent (MeOH)).

A further preferred embodiment (9) concretizing any one of embodiments (1) to (8) relates to said process for separating 1-methoxypropan-2-ol from an aqueous stream comprising 1-methoxypropan-2-ol and 2-methoxypropan-1-ol, wherein stream S0 provided in a) comprises 1-methoxypropan-2-ol and 2-methoxypropan-1-ol in a molar ratio in the range of from 1 : 4 to 4 : 1; preferably in the range of from 1 : 3 to 3 : 1, more preferred in the range of from 1 : 2 to 2 : 1, more preferred in the range of from 0.75 : 1 to 1 : 0.75.

A further preferred embodiment (10) concretizing any one of embodiments (1) to (9) relates to said process for separating 1-methoxypropan-2-ol from an aqueous stream comprising 1-methoxypropan-2-ol and 2-methoxypropan-1-ol, wherein stream S0 provided in a) comprises propylene glycol dimethyl ether in an amount in the range of from 0.001 to 0.1 weight-%, preferably in the range of from 0.003 to 0.01 weight-%, more preferred in the range of from 0.004 to 0.01 weight-%, more preferred in the range of from 0.005 to 0.008 weight-%, based on the total weight of S0.

A further preferred embodiment (11) concretizing any one of embodiments (1) to (10) relates to said process for separating 1-methoxypropan-2-ol from an aqueous stream comprising 1-methoxypropan-2-ol and 2-methoxypropan-1-ol, wherein the distillation column B comprised in the distillation unit according to b) has between 5 and 100 theoretical stages, preferably between 8 and 60 theoretical stages, more preferred between 10 and 40 theoretical stages, more preferred between 15 and 30 theoretical stages.

A further preferred embodiment (12) concretizing any one of embodiments (1) to (11) relates to said process for separating 1-methoxypropan-2-ol from an aqueous stream comprising 1-methoxypropan-2-ol and 2-methoxypropan-1-ol, wherein the distillation column B comprised in the distillation unit according to b) is operated at a pressure in the range of from 2 to 30 bar, preferably in the range of from 2.5 to 20 bar, more preferred in the range of from 3 to 15 bar, more preferred in the range of from 3.5 to 14 bar, more preferred in the range of from 4 to 12 bar, more preferred in the range of from 5 to 11 bar.

A further preferred embodiment (13) concretizing any one of embodiments (1) to (12) relates to said process for separating 1-methoxypropan-2-ol from an aqueous stream comprising 1-methoxypropan-2-ol and 2-methoxypropan-1-ol, wherein the distillation column B comprised in the distillation unit according to b) is operated at a temperature at the top of the distillation column B in the range of from 140 to 250 °C, preferably in the range of from 150 to 220 °C, more preferred in the range of from 160 to 200 °C, more preferred in the range of from 170 to 190 °C.

A further preferred embodiment (14) concretizing any one of embodiments (1) to (13) relates to said process for separating 1-methoxypropan-2-ol from an aqueous stream comprising 1-methoxypropan-2-ol and 2-methoxypropan-1-ol, wherein the distillation column B comprised in the distillation unit according to b) is operated at a temperature at the bottom in the range of from 140 to 250 °C, preferably in the range of from 150 to 220 °C, more preferred in the range of from 160 to 200 °C, more preferred in the range of from 170 to 190 °C.

A further preferred embodiment (15) concretizing any one of embodiments (1) to (14) relates to said process for separating 1-methoxypropan-2-ol from an aqueous stream comprising 1-methoxypropan-2-ol and 2-methoxypropan-1-ol, wherein the distillation column B comprised in the distillation unit according to b) is operated with a reflux ratio in the range of from 1 to 10 g/g, preferably in the range of from 2 to 9 g/g, more preferred in the range of from 3 to 8 g/g, more preferred in the range of from 4 to 6 g/g.

A further preferred embodiment (16) concretizing any one of embodiments (1) to (15) relates to said process for separating 1-methoxypropan-2-ol from an aqueous stream comprising 1-methoxypropan-2-ol and 2-methoxypropan-1-ol, wherein the distillation column B comprised in the distillation unit according to b) has an energy demand in the range of from 10 to 30 MW; wherein preferably the reboiler of distillation column B has an energy demand in the range of from 5 to 15 MW and the condenser of distillation column B has an energy demand the range of from 5 to 15 MW.

A further preferred embodiment (17) concretizing any one of embodiments (1) to (16) relates to said process for separating 1-methoxypropan-2-ol from an aqueous stream comprising 1-methoxypropan-2-ol and 2-methoxypropan-1-ol, wherein ≥ 95 weight-% of stream S1, which leaves distillation column B over the top, consist of water, 1-methoxypropan-2-ol and 2-methoxypropan-1-ol; wherein preferably stream S1 comprises water in a amount in the range of from 40 to 80 weight-%, preferably in the range of from 50 to 70 weight-%, more preferred in the range of from 55 to 65 weight-%; and a mixture of 1-methoxypropan-2-ol and 2-methoxypropan-1-ol in an amount in the range of from 20 to 60 weight-%, preferably in the range of from 30 to 50 weight-%, more preferred in the range of from 35 to 45 weight-%, each based on the total weight of stream S1, wherein preferably stream S1 comprises 1-methoxypropan-2-ol and 2-methoxypropan-1-ol in a molar ratio in the range of from 1 : 4 to 4 : 1; preferably in the range of from 1 : 3 to 3:1, more preferred in the range of from 1 : 2 to 2 : 1, more preferred in the range of from 0.75 : 1 to 1 : 0.75.

A further preferred embodiment (18) concretizing any one of embodiments (1) to (17) relates to said process for separating 1-methoxypropan-2-ol from an aqueous stream comprising 1-methoxypropan-2-ol and 2-methoxypropan-1-ol, wherein stream S1, which leaves distillation column B over the top, contains less than 0.05 weight-%, preferably less than 0.005 weight-%, more preferred less than 0.004 weight-%, more preferred less than 0.002 weight-%, of propylene glycol dimethyl ether, based on the total weight of S1.

A further preferred embodiment (19) concretizing any one of embodiments (1) to (18) relates to said process for separating 1-methoxypropan-2-ol from an aqueous stream comprising 1-methoxypropan-2-ol and 2-methoxypropan-1-ol, wherein stream S1 after leaving column B and before entering the membrane unit M is flashed [to reduce the heat demand in column C].

A further preferred embodiment (20) concretizing any one of embodiments (1) to (19) relates to said process for separating 1-methoxypropan-2-ol from an aqueous stream comprising 1-methoxypropan-2-ol and 2-methoxypropan-1-ol, wherein stream S1a comprises ≥ 95 weight-% of water, and preferably less than 1 weight-% of stream S1a consist of 1-methoxypropan-2-ol and 2-methoxypropan-1-ol, each based on the total weight of stream S1a, more preferably S1a comprises ≥ 98 weight-% of water, and less than 1 weight-% of stream S1a consist of 1-methoxypropan-2-ol and 2-methoxypropan-1-ol; more preferred S1a comprises ≥ 99 weight-% of water, and less than 1 weight-% of stream S1a consist of 1-methoxypropan-2-ol and 2-methoxypropan-1-ol.

A further preferred embodiment (21) concretizing any one of embodiments (1) to (20) relates to said process for separating 1-methoxypropan-2-ol from an aqueous stream comprising 1-methoxypropan-2-ol and 2-methoxypropan-1-ol, wherein the membrane unit M according to (c.1) comprises one or more membrane modules, each membrane module comprising at least one membrane, and optionally one or more further component selected from the group consisting of heat exchanger, pump compressor and condenser.

A further preferred embodiment (22) concretizing any one of embodiments (1) to (21) relates to said process for separating 1-methoxypropan-2-ol from an aqueous stream comprising 1-methoxypropan-2-ol and 2-methoxypropan-1-ol, wherein the membrane(s) comprised in the one or more membrane modules, which are comprised in the membrane unit M according to (c.1) comprise(s) a material selected from the group consisting of polymers; inorganic materials, preferably zeolites; carbon materials; hybrid inorganic-organic materials, preferably hybrid silica materials; and composite materials; wherein the material of each membrane may be the same or different.

A further preferred embodiment (23) concretizing any one of embodiments (1) to (22) relates to said process for separating 1-methoxypropan-2-ol from an aqueous stream comprising 1-methoxypropan-2-ol and 2-methoxypropan-1-ol, wherein the membrane unit M according to (c.1) is operated in pervaporation mode or vapor permeation mode, preferably in pervaporation mode.

A further preferred embodiment (24) concretizing any one of embodiments (1) to (23) relates to said process for separating 1-methoxypropan-2-ol from an aqueous stream comprising 1-methoxypropan-2-ol and 2-methoxypropan-1-ol, wherein the one or more membrane module(s) of the membrane unit M according to (c.1) are operated under pervaporation conditions and are arranged in loops that preferably comprise, more preferred consist of, at least a crossflow pump, a heat exchanger, and one or more membrane modules; wherein the number of loops is preferably in the range of from 1 to 10 loops, preferably in the range of from 1 to 4 loops, more preferred in the range of from 2 to 3 loops.

A further preferred embodiment (25) concretizing any one of embodiments (1) to (24) relates to said process for separating 1-methoxypropan-2-ol from an aqueous stream comprising 1-methoxypropan-2-ol and 2-methoxypropan-1-ol, wherein the one or more membrane module(s) of the membrane unit M according to (c.1) are operated under pervaporation conditions and are connected in series with interstage heat exchangers.

A further preferred embodiment (26) concretizing any one of embodiments (1) to (25) relates to said process for separating 1-methoxypropan-2-ol from an aqueous stream comprising 1-methoxypropan-2-ol and 2-methoxypropan-1-ol, wherein the one or more membrane modules of the membrane unit M according to (c.1) operate at a temperature in the range of from 70 to 160 °C, preferably in the range of from 90 to 140 °C, more preferred in the range of from 110 to 130 °C.

A further preferred embodiment (27) concretizing any one of embodiments (1) to (26) relates to said process for separating 1-methoxypropan-2-ol from an aqueous stream comprising 1-methoxypropan-2-ol and 2-methoxypropan-1-ol, wherein the permeate sides of one or more membrane modules of the membrane unit M according to (c.1) are operated at a vacuum pressure in the range of from 10 mbar(a) to atmospheric pressure, preferably in the range of from 20 to 200 mbar(a), more preferred in the range of from 30 to 80 mbar(a), and/or, preferably and, the retentate sides of one or more membrane modules of the membrane unit M according to (c.1) are operated at a pressure in the range of from 0.5 to 10 bar, preferably in the range of from 1 to 5 bar, more preferred in the range of from 1.5 to 2.5 bar, wherein pressure at the retentate side is higher than the pressure at the permeate side.

A further preferred embodiment (29) concretizing any one of embodiments (1) to (28) relates to said process for separating 1-methoxypropan-2-ol from an aqueous stream comprising 1-methoxypropan-2-ol and 2-methoxypropan-1-ol, wherein in the range of from 55 to 98 weight-% of S2 consists of 1-methoxy-2-propanol and 2-methoxy-1-propanol based on the total weight of S2, wherein preferably stream S2 comprises 1-methoxypropan-2-ol and 2-methoxypropan-1-ol in a molar ratio in the range of from 1 : 4 to 4 : 1; preferably in the range of from 1 : 3 to 3 : 1, more preferred in the range of from 1 : 2 to 2 : 1, more preferred in the range of from 0.75 : 1 to 1 : 0.75.

A further preferred embodiment (30) concretizing any one of embodiments (1) to (29) relates to said process for separating 1-methoxypropan-2-ol from an aqueous stream comprising 1-methoxypropan-2-ol and 2-methoxypropan-1-ol, wherein the stream S2 comprises water in the range of from 0.1 to 45 weight-%, preferably in the range of from 0.5 to 10 weight-%, more preferred in the range of from 3 to 5 weight-%, more preferred in the range of from 3.5 to 4.5 weight-%, based on the total weight of S2.

A further preferred embodiment (31) concretizing any one of embodiments (1) to (30) relates to said process for separating 1-methoxypropan-2-ol from an aqueous stream comprising 1-methoxypropan-2-ol and 2-methoxypropan-1-ol, wherein stream S2 after leaving the membrane unit M and before entering column C is flashed [to reduce the heat demand in column C].

A further preferred embodiment (32) concretizing any one of embodiments (1) to (31) relates to said process for separating 1-methoxypropan-2-ol from an aqueous stream comprising 1-methoxypropan-2-ol and 2-methoxypropan-1-ol, wherein the stream S2a is obtained from membrane unit M, wherein 90 weight-%, more preferred ≥ 95 weight-%, more preferred ≥97 weight-%, ≥ 98 weight-%, more preferred ≥ 99 weight-% of S2a consisted of water based on the total weight of S2a.

A further preferred embodiment (33) concretizing any one of embodiments (1) to (32) relates to said process for separating 1-methoxypropan-2-ol from an aqueous stream comprising 1-methoxypropan-2-ol and 2-methoxypropan-1-ol, wherein the distillation column C comprised in the distillation unit according to (c.2) has between 2 and 50 theoretical stages, preferably between 5 and 25 theoretical stages, more preferred between 10 and 20 theoretical stages.

A further preferred embodiment (34) concretizing any one of embodiments (1) to (33) relates to said process for separating 1-methoxypropan-2-ol from an aqueous stream comprising 1-methoxypropan-2-ol and 2-methoxypropan-1-ol, wherein the distillation column C comprised in the distillation unit according to (c.2) is operated at a pressure in the range of from 0.5 to 10 bar, preferably in the range of from 1 to 5 bar, more preferred in the range of from 1.5 to 2.5 bar.

A further preferred embodiment (35) concretizing any one of embodiments (1) to (34) relates to said process for separating 1-methoxypropan-2-ol from an aqueous stream comprising 1-methoxypropan-2-ol and 2-methoxypropan-1-ol, wherein the distillation column C comprised in the distillation unit according to (c.2) is operated at a bottoms temperature in the range of from 120 to 180 °C, preferably in the range of from 130 to 170 °C, more preferred in the range of from 140 to 160 °C.

A further preferred embodiment (36) concretizing any one of embodiments (1) to (35) relates to said process for separating 1-methoxypropan-2-ol from an aqueous stream comprising 1-methoxypropan-2-ol and 2-methoxypropan-1-ol, wherein the distillation column C comprised in the distillation unit according to (c.2) is operated at a temperature at the top of the column in the range of from 70 to 110 °C, preferably in the range of from 80 to 100 °C, more preferred in the range of from 85 to 95 °C.

A further preferred embodiment (37) concretizing any one of embodiments (1) to (36) relates to said process for separating 1-methoxypropan-2-ol from an aqueous stream comprising 1-methoxypropan-2-ol and 2-methoxypropan-1-ol, wherein the distillation column C comprised in the distillation unit according to (c.2) is operated with a reflux ratio in the range of from 10 to 40 g/g, preferably in the range of from 12 to 30 g/g, more preferred in the range of from 15 to 25 g/g.

A further preferred embodiment (38) concretizing any one of embodiments (1) to (37) relates to said process for separating 1-methoxypropan-2-ol from an aqueous stream comprising 1-methoxypropan-2-ol and 2-methoxypropan-1-ol, wherein in step (e) at least 50 weight-% of S3a, preferably at least 60 weight-% of S3a, more preferred at least 70 weight-% of S3a, more preferred at least 80 weight-% of S3a, more preferred at least 90 weight-% of S3a, more preferred at least 95 weight-% of S3a, more preferred at least 99 weight-% of S3a, more preferred at least 99.5 weight-% of S3a, more preferred at least 100 weight-% of S3a, are recycled to the membrane unit M of (c.1).

A further preferred embodiment (39) concretizing any one of embodiments (1) to (38) relates to said process for separating 1-methoxypropan-2-ol from an aqueous stream comprising 1-methoxypropan-2-ol and 2-methoxypropan-1-ol, wherein stream S3a comprises water, 1-methoxypropan-2-ol and 2-methoxypropan-1-ol, wherein preferably S3a comprises less than 10 weight-% of water, based on the total weight of S3a, and/or more than 80 weight-% of S3a are 1-methoxypropan-2-ol and 2-methoxypropan-1-ol.

A further preferred embodiment (40) concretizing any one of embodiments (1) to (39) relates to said process for separating 1-methoxypropan-2-ol from an aqueous stream comprising 1-methoxypropan-2-ol and 2-methoxypropan-1-ol, wherein stream S3a comprises ≥ 70 weight-%, preferably ≥ 75 weight-%, more preferred ≥ 80 weight-%, 2-methoxypropan-1-ol, based on the total weight of S3a; and/or wherein stream S3a comprises ≤ 20 weight-%, preferably ≤ 15 weight-%, more preferred ≤ 10 weight-%, 1-methoxypropan-2-ol, based on the total weight of S3a.

A further preferred embodiment (41) concretizing any one of embodiments (1) to (40) relates to said process for separating 1-methoxypropan-2-ol from an aqueous stream comprising 1-methoxypropan-2-ol and 2-methoxypropan-1-ol, wherein stream S3 comprises water, 1-methoxypropan-2-ol and 2-methoxypropan-1-ol, wherein S3 preferably comprises ≤ 1 weight-%, more preferred ≤ 0.1 weight-%, water, based on the total weight of S3.

A further preferred embodiment (42) concretizing any one of embodiments (1) to (41) relates to said process for separating 1-methoxypropan-2-ol from an aqueous stream comprising 1-methoxypropan-2-ol and 2-methoxypropan-1-ol, wherein stream S3 comprises ≥95 weight-%, preferably ≥96 weight-%, more preferred ≥97 weight-%, more preferred ≥98 weight-%, more preferred ≥99 weight-%, of 1-methoxypropan-2-ol and 2-methoxypropan-1-ol, based on the total weight of S3; and/or, preferably and, wherein S3 has a water content of at most 100 ppm, based on the total weight of S3.

A further preferred embodiment (43) concretizing any one of embodiments (1) to (42) relates to said process for separating 1-methoxypropan-2-ol from an aqueous stream comprising 1-methoxypropan-2-ol and 2-methoxypropan-1-ol, wherein stream S3 comprises 1-methoxypropan-2-ol and 2-methoxypropan-1-ol in a molar ratio in the range of from 1 : 4 to 4 : 1; preferably in the range of from 1 : 3 to 3 : 1, more preferred in the range of from 1 : 2 to 2 : 1, more preferred in the range of from 0.75 : 1 to 1 : 0.75

A further preferred embodiment (44) concretizing any one of embodiments (1) to (43) relates to said process for separating 1-methoxypropan-2-ol from an aqueous stream comprising 1-methoxypropan-2-ol and 2-methoxypropan-1-ol, wherein the distillation column D comprised in the distillation unit according to (d) has between 20 and 100 theoretical stages, preferably between 30 and 80 theoretical stages, more preferred between 40 and 60 theoretical stages, more preferred between 45 and 52 theoretical stages.

A further preferred embodiment (45) concretizing any one of embodiments (1) to (44) relates to said process for separating 1-methoxypropan-2-ol from an aqueous stream comprising 1-methoxypropan-2-ol and 2-methoxypropan-1-ol, wherein the distillation column D comprised in the distillation unit according to (d) is operated at a pressure in the range of from 0.5 to 10 bar, preferably in the range of from 1 to 5 bar, more preferred in the range of from 2 to 4 bar.

A further preferred embodiment (46) concretizing any one of embodiments (1) to (45) relates to said process for separating 1-methoxypropan-2-ol from an aqueous stream comprising 1-methoxypropan-2-ol and 2-methoxypropan-1-ol, wherein the distillation column D comprised in the distillation unit according to (d) is operated at a bottoms temperature in the range of from 140 to 200 °C, preferably in the range of from 150 to 190 °C, more preferred in the range of from 160 to 180 °C.

A further preferred embodiment (47) concretizing any one of embodiments (1) to (46) relates to said process for separating 1-methoxypropan-2-ol from an aqueous stream comprising 1-methoxypropan-2-ol and 2-methoxypropan-1-ol, wherein the distillation column D comprised in the distillation unit according to (d) is operated at a temperature at the top of the column in the range of from 120 to 200 °C, preferably in the range of from 130 to 180 °C, more preferred in the range of from 140 to 165 °C.

A further preferred embodiment (48) concretizing any one of embodiments (1) to (47) relates to said process for separating 1-methoxypropan-2-ol from an aqueous stream comprising 1-methoxypropan-2-ol and 2-methoxypropan-1-ol, wherein the distillation column D comprised in the distillation unit according to (d) is operated with a reflux ratio in the range of from 5 to 30 g/g, preferably in the range of from 8 to 20 g/g, more preferred in the range of from 10 to 15 g/g.

A further preferred embodiment (49) concretizing any one of embodiments (1) to (48) relates to said process for separating 1-methoxypropan-2-ol from an aqueous stream comprising 1-methoxypropan-2-ol and 2-methoxypropan-1-ol, wherein the distillation column D comprised in the distillation unit according to (d) has an energy demand in the range of from 1 to 5 MW; wherein preferably the reboiler of distillation column D has an energy demand in the range of from 1 to 1.5 MW and the condenser of distillation column D has an energy demand the range of from 1 to 1.5 MW.

A further preferred embodiment (50) concretizing any one of embodiments (1) to (49) relates to said process for separating 1-methoxypropan-2-ol from an aqueous stream comprising 1-methoxypropan-2-ol and 2-methoxypropan-1-ol wherein the stream S4 is removed as bottoms stream from distillation column D, wherein S4 comprises ≥ 95 weight-% 2-methoxypropan-1-ol and ≤ 0.5 weight- % 1-methoxypropan-2-ol, preferably ≥ 96 weight-% 2-methoxypropan-1-ol and ≤ 0.1 weight- % 1-methoxypropan-2-ol, more preferred ≥ 98 weight-% 2-methoxypropan-1-ol and ≤ 0.001 weight-% 1-methoxypropan-2-ol, more preferred ≥ 99 weight-% 2-methoxypropan-1-ol and ≤ 0.0001 weight- % 1-methoxypropan-2-ol, each based on the total weight of stream S4.

A further preferred embodiment (51) concretizing any one of embodiments (1) to (50) relates to said process for separating 1-methoxypropan-2-ol from an aqueous stream comprising 1-methoxypropan-2-ol and 2-methoxypropan-1-olwherein the stream S5 is removed as top stream from distillation column D, which comprises ≥ 95 weight-% 1-methoxypropan-2-ol and ≤ 0.5 weight-% of 2-methoxypropan-1-ol, preferably ≥ 98 weight-% 1-methoxypropan-2-ol and ≤ 0.15 weight-% of 2-methoxypropan-1-ol, more preferred ≥ 99 weight-% 1-methoxypropan-2-ol and ≤ 0.1 weight- % of 2-methoxypropan-1-ol, each based on the total weight of stream S5.

A further preferred embodiment (52) concretizing any one of embodiments (1) to (51) relates to said process for separating 1-methoxypropan-2-ol from an aqueous stream comprising 1-methoxypropan-2-ol and 2-methoxypropan-1-ol wherein the stream S5 comprises ≤ 0.01 weight-%, preferably ≤ 0.008 weight-%, more preferred ≤ 0.007 weight-%, more preferred ≤ 0.006 weight-%, of propylene glycol dimethyl ether based on the total weight of stream S5.

The present invention is further illustrated by the following reference examples, comparative examples, and examples.

**Examples**

**Simulations**

All simulations were done with process simulation software Aspen Plus v.11. The components used in the process simulation and their characteristics respectively, were taken from the Dortmund Database.

**Example 1:** **separation of 1-methoxypropan-2-ol from an aqueous stream (S0) containing 85 weight-% of water and 14.2 weight-% of a mixture of 2-methoxypropan-1-ol and 1-methoxypropan-2-ol - distillation column B operated at ≥ 2 bar**

The feed stream S0 to column B was a variable stream and represented a stream from a propylene oxide production process, wherein a reaction mixture comprising propylene, water, methanol, and hydrogen peroxide had been contacted in an epoxidation zone with an epoxidation catalyst comprising a zeolitic material having a framework structure comprising Si, O, and Ti and being of framework type MFI (titanium silicalite-1 (TS-1)), and the reaction mixture had been subjected to epoxidation reaction conditions in the epoxidation zone.

The obtained mixture comprising propylene oxide, water, and methanol had been removed as an effluent stream from the epoxidation zone. The effluent stream comprising propylene oxide, water, and methanol had been subjected to further separation and purification steps, wherein propylene oxide and water as well as parts of the organic solvent had been removed, resulting in a stream S0 comprising 1-methoxypropan-2-ol, 2-methoxypropan-1-ol and water and having a molar ratio of 1-methoxypropan-2-ol : 2-methoxypropan-1-ol in the range of from 1 : 5 to 5 : 1. The composition of stream S1 varied as a function of the operating conditions of the propylene oxide production process . An exemplary composition of stream S0,exemplary compositions of the further streams S1 to S5 are indicated in Table 1a in view of water, 1-methoxypropan-2-ol and 2-methoxypropan-1-ol and physical parameters of these streams, Table 1b shows the energy demand of the reboilers and condensers as well as the respective temperature ranges. In Table 2, compositions of streams S0, S1 and S1a are listed. The expression "E-xx" in Tables 1a and 2 represents $10^{-xx}$, wherein "xx" is here a placeholder for the respective number indicated in the Tables. Stream S3a was completely recirculated back to the membranes. Therefore, the feed stream to the membrane unit M is the combination of stream S1 and stream S3a.

**Table 1a**

| Compositions and physical parameters of streams S0 to S5 as well as energy demand of components | | | | | | | | | | |
|---|---|---|---|---|---|---|---|---|---|---|
| | S0 | S1a | S1 | S2a (after condensation) | S2 | S2 (S2 after heating up) Feed to column C | S3a | S3 | S5 | S4 |
| Temperature [°C] | 181 | 179.9 | 175 | 38.9 | 115 | 170 | 109 | 148 | 154 | 172.1 |
| Pressure [bar] | 10 | 10 | 10 | 0.07 | 10 | 10 | 2 | 2 | 3 | 3 |
| Mass flow [kg/h] | 11415 | 7371 | 4044 | 2481 | 1779 | 1779 | 215.4 | 1558 | 754.9 | 803.1 |
| Concentrations in weight-% (unless indicated otherwise) | | | | | | | | | | |
| Water | 85.79 | 99.11 | 60.1 | 97.88 | 4 | 4 | 31.884 | 100 ppm | 206 ppm | 0 |
| 1-Methoxypropan-2-ol | 6.95 | 9.42E-03 | 19.43 | 1.12 | 50.55 | 50.55 | 65.56 | 48.45 | 99.95 | 500 ppm |
| 2-Methoxypropan-1-ol | 7.26 | 5.83E-04 | 20.48 | 1.01 | 45.45 | 45.45 | 2.55 | 51.54 | 300 ppm | 99.95 |

**Table 1b**

| Energy demand of condensers and reboilers as well as temperature ranges of these components. | | | | | | | | | | |
|---|---|---|---|---|---|---|---|---|---|---|
| | Condenser column B | Reboiler column B | Condenser column C | Reboiler column C | Condenser column D | Reboiler column D | W100 | W200 | Wflash | W300 |
| Thermal energy demand [kW] | 9180 | 8900 | 232.47 | 197.14 | 1100 | 1121 | 1079.54 | 181.32 | 78.2 | 1728.73 |
| Temperature range [°C] | 177-175 | 179.9-181 | 119-109 | 148-156 | 156-154 | 172-174 | 115-120 | 115-120 | 115-170 | 115-38 |

**Table 2**

| compositions of streams S0, S1a and S1. | | | |
|---|---|---|---|
| Streams | S0 | S1a | S1 |
| Parameter | | | |
| Temperature [°C] | 181 | 179.927 | 175.099 |
| Pressure [bar] | 10 | 10 | 10 |
| Mass flow [kg/h] | 11415 | 7371 | 4044 |
| Components, indicated in weight-% | | | |
| **2-Methoxypropan-1-ol** | **7.26** | **5.83E-04** | **20.48** |
| Water | 85.17 | 99.11 | 59.98 |
| Benzene | | | |
| **1-Methoxypropan-2-ol** | **6.95** | **9.42E-03** | **19.46** |
| 1,1-Dimethoxyethane | | | |
| 1,1-Dimethoxypropane | 1.00E-03 | | 1.34E-03 |
| 1,2-Propanediol (MPG) | 1.00E-02 | 1.55E-02 | 9.45E-08 |
| 1-Butanol | 1.00E-03 | | 2.27E-03 |
| 2,4-Dimethyl-1,3-dioxolane | 1.00E-03 | | 1.81E-03 |
| 2,6-Dimethyl-4-heptanol | 1.00E-03 | | 1.71E-03 |
| 2-Butenal | 1.00E-03 | | 2.22E-03 |
| 2-Ethyl-4-methyl-1,3-dioxolane | 1.00E-03 | 1.55E-03 | 5.20E-06 |
| 2-Hexanone | 1.00E-01 | | 1.56E-01 |
| 2-Methylcyclohexanol | 1.00E-03 | 1.55E-03 | 5.20E-06 |
| 2-Methylpentanal | 4.00E-03 | | 4.85E-03 |
| 2-Propen-1-ol | 1.00E-03 | | 2.51E-03 |
| 4-Methyl-1,3-dioxolane | 1.00E-03 | 3.53E-10 | 2.76E-03 |
| Acetaldehyde | 1.00E+00 | | 1.46E-03 |
| Acetone | 1.00E-03 | | 2.06E-03 |
| Dimethoxymethane | 1.00E-03 | | 1.99E-03 |
| Dipropyleneglycol (DPG) | 2.00E-02 | 3.10E-02 | 1.04E-04 |
| Ethanol | 1.00E-03 | | 2.28E-03 |
| Hydroxyacetone | 1.00E-03 | 1.51E-03 | 7.55E-05 |
| 2-Propanol | 1.00E-03 | | 2.03E-03 |
| Methanol | 1.99E-02 | | 4.84E-02 |
| Methylacetate | 1.00E-03 | | 1.68E-03 |
| Methylformate | 1.00E-03 | 1.54E-03 | 1.81E-05 |
| Propyleneoxide | 1.00E-03 | | 1.59E-03 |
| Tripropylene glycol (TPG) | 2.00E-02 | 3.10E-02 | 7.75E-10 |
| Dipropylene glycol mono methyl ether (DPGME) | 5.10E-01 | 7.90E-01 | 4.63E-06 |
| **Propylene glycol dimethyl ether (1,2-Dimethoxypropane)** | **6.50E-03** | **9.23E-03** | **1.56E-03** |

Column B was a pre-distillation column, used to enrich the mixture of 1-methoxy-2-propanol and 2-methoxy-1-propanol contained in stream S0. In column B, the mixture of 1-methoxy-2-propanol and 2-methoxy-1-propanol was separated from side components. in order to ensure that the final product 1-methoxy-2-propanol has a purity of > 95 weight-%, preferably ≥ 98 weight-%, more preferred ≥ 99 weight-%, more preferred ≥ 99.7 weight-%. Column B had 20 theoretical stages and was operated at 10 bar. Feed stream S0 entered the column B at theoretical stage 17 (between stage 17 and 18). The temperature at the top was 177 °C and at the bottom 180 °C. Column B was operated with a reflux ratio of 4.93 g/g and 8900 kW were needed in the reboiler and 9180 kW in the condenser. An azeotropic mixture of water, 1-methoxy-2-propanol and 2-methoxy-1-propanol was removed from column B over the top (stream S1: about 60 weight-% water, 40 weight-% mixture of 1-methoxy-2-propanol and 2-methoxy-1-propanol). Negligible amounts of side components were removed from column B as bottoms stream S1a, which was afterwards send to a subsequent water treatment or recycled into one or more stages of the process or recycled into one or more stages of an upstream HPPO process, from which stream S0 derives, preferably S1a is sent to water treatment or recycled into one or more stages of an upstream HPPO process, from which stream S0 derives.

Membrane unit M

Stream S1, was, together with stream S3a, transferred to membrane unit M operated at 10 bar, which consisted of two membrane loops in series, each comprising at least one membrane module. In the first loop, the membrane surface area was 120 m$^2$. In the second loop, the membrane surface area was 120 m$^2$ as well. In both loops, the crossflow over the membranes was adjusted to such a value that the temperature drop over the membrane module was 5 °C. Both loops operated at a permeate pressure of 0.07 bar using a combined vacuum system. In the membrane unit M, water was separated resulting in a stream S2 having a temperature of 115 °C. Stream S2 was flashed before entering Column C in a further flash column, where the pressure was reduced from 10 bar to 2 bar. This decreased the temperature of stream S2.

The water flux (Flux(water)) through the membrane was calculated by the following equation 1:

$$Flux(water) = 217.57 \times (W_{H20,RET})^3 - 241.41 \times \left(W_{H20,RET}\right)^2 + 155.39 \, x \left(W_{H20,RET}\right) - 2.5977$$

-equation 1-

wherein $W_{H2O,RET}$ is the water concentration (indicated in g water / g total solution) in the retentate.

The flux of both methoxypropanols (MOP) 1-methoxy-2-propanol and 2-methoxy-1-propanol (Flux(MOP)) through the membrane was calculated by the following equation 2:

$$Flux(MOP) = 15.374 \times (W_{H20,RET})^3 - 7.6276 \times \left(W_{H20,RET}\right)^2 + 2.3173 \, x \left(W_{H20,RET}\right) + 0.06$$

-equation 2-

wherein $W_{H2O,RET}$ is the water concentration (indicated in g water / g total solution) in the retentate.

In principle, 1-methoxy-2-propanol and 2-methoxy-1-propanol each has its own permeability but the respective values differ from each other by less than 5%. Thus, it was assumed for the above-indicated equation 2 that the permeabilities of the two MOPs 1-methoxy-2-propanol and 2-methoxy-1-propanol are identical. Stream S2 was heated to a temperature of 170 °C and then transferred to Column C at theoretical stage 8, wherein column C was a distillation column with 16 theoretical stages operated at a pressure of 2 bar. Column C had a temperature at the top of 109 °C and at the bottom of 148°C. Column C was operated with a reflux ratio of 2.93 g/g and 197.14 kW were needed in the reboiler and 232.47 kW in the condenser. A bottom streams S3 was removed from Column C, wherein ≥ 85 weight-% of S2 consisted of 1-methoxy-2-propanol and 2-methoxy-1-propanol. A stream S3a consisting to more than 90 weight-% of water was removed from column C over the top and condensed in a condenser unit. Stream S3a in condensed form was recycled to membrane unit M. Stream S3 was transferred to column D, which was a distillation column with 48 theoretical stages operated at 3 bar. The temperature at the top of column D was 156 °C and at the bottom 172 °C. Column D was operated with a reflux ratio of 12.2 g/g and 1121 kW were needed in the reboiler and 1100 kW in the condenser. From column D, a stream S5 was removed over the top comprising, 99.95 weight-% 1-methoxylpropan-2-ol based on the total weight of stream S5 and having an isolation yield of 95.1 %. As bottoms streams, a stream S4 was removed, wherein S4 comprised 99.95 weight-% 2-methoxylpropan-1-ol based on the weight of S4 and having an isolation yield of 96.85. It could be seen that propylene glycol dimethyl was effectively separated: Already in S1, the content of propylene glycol dimethyl was only 1.56 x $10^{-3}$ weight-% based on the weight of S1. Stream S5 contained only 5.25 x $10^{-3}$ weight-% propylene glycol dimethyl ether based on the total weight of S5.

**Heat integration**

A very important part of the separation process in including columns B to D and membrane unit M was the heat integration, because it reduces significantly the investment costs in terms of steam. For the heat integration there were several possibilities, two thereof were simulated:

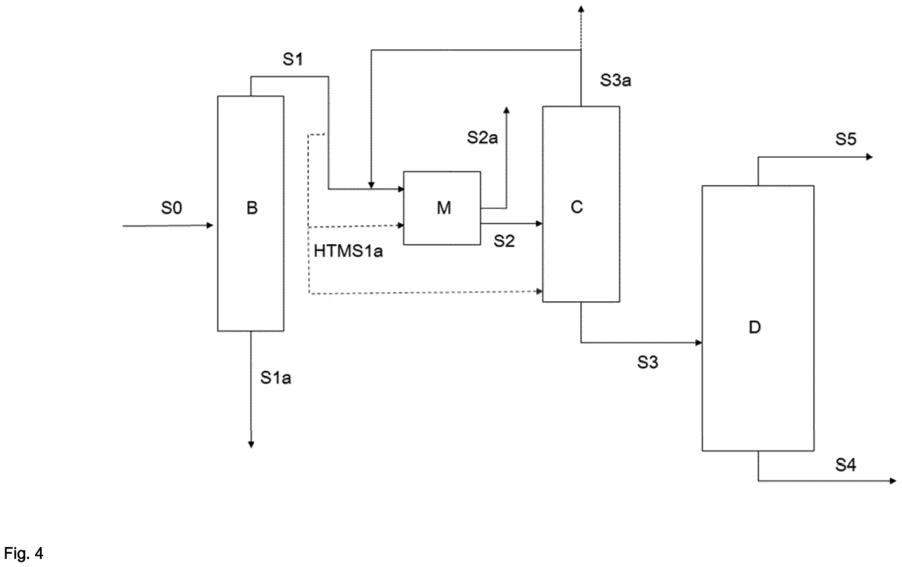

1. as shown in Fig. 4, the thermal energy (heat) of the condenser of column B, i.e. of stream S1, was used to heat the reboiler of column C and the membrane unit M in that thermal energy of stream S1 was partly transferred to a heat transfer medium stream HTMS1 before entering the membrane unit M in a heat exchanger H, wherein a heat transfer medium stream HTMS1a was obtained which had an increased thermal energy content compared to HTMS1. The heat transfer medium stream HTMS1a was used to provide thermal energy to a heat exchanger unit, which supplied the reboiler of column C and to the reboiler of membrane unit M. The total heat demand was 9600 kW.

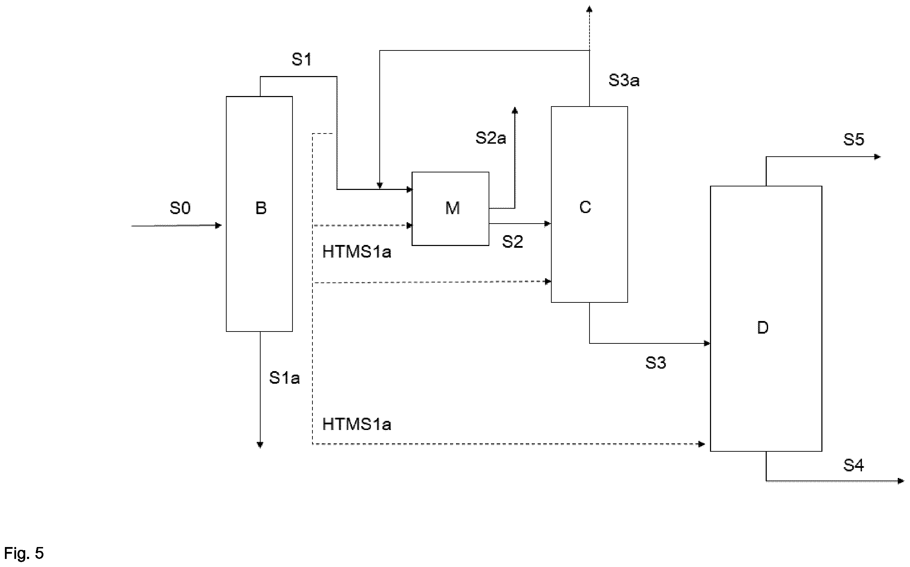

2. as shown in Fig. 5, a complete heat integration was made. The thermal energy (heat) of the condenser of column B, i.e. of stream S1, was used to heat the reboiler of columns C and D in that thermal energy of stream S1 was partly transferred to a heat transfer medium stream HTMS1 before entering membrane unit M in a heat exchanger H, wherein a heat transfer medium stream HTMS1a was obtained which had an increased thermal energy content compared to HTMS1. The heat transfer medium stream HTMS1a was used to provide thermal energy to a heat exchanger unit, which supplied the reboiler of column C, to the reboiler of membrane unit M and to heat exchanger unit, which supplied the reboiler of column D. The total energy demand was 8900 kW.

The heat transfer medium of HTMS1/HTMS1a and of HTMS2/HTMS2a was steam ($H_2O_{gaseous}$). This example demonstrates that 1-methoxypropanol-2 with a purity of 99.95% can be obtained with an isolation yield of 95.1 %. Additionally, 2-methoxypropanol-1 with a purity of 99.95 % can be obtained with an isolation yield of 96.85 %.

**Comparative Example 1:    separation of 1-methoxypropan-2-ol from an aqueous stream (S0) containing 85 weight-% of water and 14.2 weight-% of a mixture of 2-methoxypropan-1-ol and 1-methoxypropan-2-ol - distillation column B operated at < 2 bar**

Herein, the same set-up with the same columns B, C and D and membrane unit M as in Example 1 was used, the only difference to Example 1 was that in column B a pressure of 1 bar was used and in columns C and D a pressure of also 1 bar was used. As in Example 1, stream S3a was completely recirculated back to the membranes. Therefore, the feed stream to the membrane unit M is the combination of stream S1 and stream S3a.

Having 1 bar in column B influenced the pressure of the rest of the columns in the process. In Example 1, column B was designed for 10 bar. Here, operating column B at 1 bar made the separation more difficult and no heat integration possible due to the lower temperatures reached in column B. In column B, more water/MOPs azeotrope went over the top (stream S1) that needed to be separated in the membranes of membrane unit M or in column C. The amount of water in S2 was fixed to 4 weight-%, which implies that the membrane area had to be larger to remove more water. Table 3a shows the composition of streams S0 and S1 to S5 and physical parameters of these streams, Table 3b lists the energy demand in condensers and reboilers and operation temperature ranges of these components. In Table 4, the complete compositions of streams S0, S1a and S1 are indicated.

**Table 3a**

| composition of streams S0 and S1 to S5 and physical parameters of these streams | | | | | | | | | | |
|---|---|---|---|---|---|---|---|---|---|---|
| | S0 | S1a | S1 | S2a (after condensation) | S2 | S2 (after heating up) Feed to column C | S3a | S3 | S5 | S4 |
| Temperature [°C] | 181 | 99.66 | 95.29 | 38.93 | 115 | 170 | 79.45 | 124.75 | 115.9 | 131.42 |
| Pressure [bar] | 1 | 1 | 1 | 0.07 | 1 | 1 | 1 | 1 | 1 | 1 |
| Mass flow [kg/h] | 11415 | 5394.80 | 6020.19 | 4473 | 1800 | 1800 | 252.40 | 1542 | 745.60 | 796.40 |
| Concentrations in weight-% (unless indicated otherwise) | | | | | | | | | | |
| Water | 85.79 | 99.8 | 72.87 | 98.03 | 4 | 4 | 27.67 | 100 ppm | 207 ppm | 0 |
| 1-Methoxypropan-2-ol | 6.95 | 5.13E-03 | 13.22 | 1.05 | 51.36 | 51.3604 | 69.58 | 48.36 | 99.95 | 500 ppm |
| 2-Methoxypropan-1-ol | 7.26 | 4.87E-03 | 13.91 | 0.92 | 44.64 | 44.6396 | 2.75 | 51.63 | 300 ppm | 99.95 |

**Table 3b**

| Energy demand in condensers and reboilers and operation temperature ranges of these components. | | | | | | | | | | |
|---|---|---|---|---|---|---|---|---|---|---|
| | Condenser column B | Reboiler column B | Condenser column C | Reboiler column C | Condenser column D | Reboiler column D | W100 | W200 | Wflash | W300 |
| Thermal Energy demand [kW] | 18005.9 | 16930 | 287.74 | 213.83 | 1411 | 1412 | 2587.4 | 272.1 | 79.18 | 3120.5 |
| Temperature range [°C] | 98.7-95.29 | 99.66-102.35 | 83-79.45 | 124.75-138 | 119-115.9 | 131.42-134 | 115-120 | 115-120 | 115-170 | 115-38 |
| Membrane area PV100 [m$^2$] | 178.57 | | | | | | | | | |
| Membrane area PV200 [m$^2$] | 178.57 | | | | | | | | | |

**Table 4**

| compositions of streams S0, S1a and S1. | | | |
|---|---|---|---|
| **Streams** | **S0** | **S1a** | **S1** |
| Parameter | | | |
| Temperature [°C] | 181 | 99.66 | 95.29 |
| Pressure [bar] | 1 | 1 | 1 |
| Mass flow [kg/h] | 11415 | 5394.81 | 6020.20 |
| Components, indicated in weight-% | | | |
| **2-Methoxypropan-1 -ol** | 7.26 | 1.00E-02 | 13.87 |
| Water | 85.17 | 98.8 | 72.87 |
| Benzene | | | |
| **1-Methoxypropan-2-ol** | 6.95 | 2.46E-06 | 13.18 |
| 1,1-Dimethoxyethane | | | |
| 1,1-Dimethoxypropane | 1.00E-03 | | 7.58E-04 |
| 1,2-Propanediol (MPG) | 1.00E-02 | 2.10E-02 | 2.09E-09 |
| 1-Butanol | 1.00E-03 | | 1.81E-03 |
| 2,4-Dimethyl-1,3-dioxolane | 1.00E-03 | | 1.36E-03 |
| 2,6-Dimethyl-4-heptanol | 1.00E-03 | | 1.50E-03 |
| 2-Butenal | 1.00E-03 | | 1.72E-03 |
| 2-Ethyl-4-methyl-1,3-dioxolane | 1.00E-03 | 2.09E-03 | 1.26E-05 |
| 2-Hexanone | 1.00E-01 | | 1.39E-03 |
| 2-Methylcyclohexanol | 1.00E-03 | 2.09E-03 | 1.26E-05 |
| 2-Methylpentanal | 4.00E-03 | | 4.10E-03 |
| 2-Propen-1-ol | 1.00E-03 | | 1.83E-03 |
| 4-Methyl-1,3-dioxolane | 1.00E-03 | | 1.87E-03 |
| Acetaldehyde | 1.00E+00 | | 1.21E-03 |
| Acetone | 1.00E-03 | | 1.61E-03 |
| Dimethoxymethane | 1.00E-03 | | 1.22E-03 |
| Dipropyleneglycol (DPG) | 2.00E-02 | 4.17E-02 | 2.52E-04 |
| Ethanol | 1.00E-03 | | 1.82E-03 |
| Hydroxyacetone | 1.00E-03 | 1.75E-03 | 3.16E-04 |
| 2-Propanol | 1.00E-03 | | 1.76E-03 |
| Methanol | 1.99E-02 | | 3.66E-02 |
| Methylacetate | 1.00E-03 | | 1.36E-02 |
| Methylformate | 1.00E-03 | | 1.35E-03 |
| Propyleneoxide | 1.00E-03 | | 1.24E-03 |
| Tripropylene glycol (TPG) | 2.00E-02 | 4.20E-02 | |
| Tripropylene glycol mono methyl ether (DPGME) | 5.10E-01 | 1.07E+00 | 5.13E-07 |
| Dipropylene glycol mono methyl ether (DPGME) | | | |

(continued)

| | | | |
|---|---|---|---|
| 1,1-Dimethoxyethane | | | |
| **Propylene glycol dimethyl ether (1,2-Dimethoxypropane)** | 6.50E-03 | 3.56E-03 | 9.18E-03 |

The total thermal energy needed in the reboiler of column B was 16930 kW, higher than in example 1 when column B was operated at 10 bar. This is because the separation was more difficult when column B was operated at a pressure below 2 bar. No heat integration was possible, i.e. the total energy demand was 21494.51 kW.

Stream S5 contained 99.95 weight-% 1-methoxypropan-2-ol based on the total weight of S5 and the isolation yield was 93.93%. Stream S4, contained 99.95 weight-% 2-methoxypropan-1-oland the isolation yield was 96.05% To achieve 4 weight-% water in S2, each membrane had to have an area of 178.57 $m^2$, the total membrane area was thus 357.14 $m^2$.

Also the impurity content in the final stream S5 was higher when column B was operated at 1 bar: Already in S1, the content of propylene glycol dimethyl was $9.18 \times 10^{-3}$ weight-% based on the weight of S1 compared to Example 1, where the content of propylene glycol dimethyl was only $1.56 \times 10^{-3}$ weight-% based on the weight of S1. The propylene glycol dimethyl ether content in S5, based on the total weight of S5, was 0.0482 weight-% compared to only $5.52 \times 10^{-3}$ weight-% as in Example 1. That is, operating column B, and, consequently, also the further column C at 1 bar did not allow to have less than 0.006 weight-% of propylene glycol dimethyl ether in S5 based on the total weight of S5.

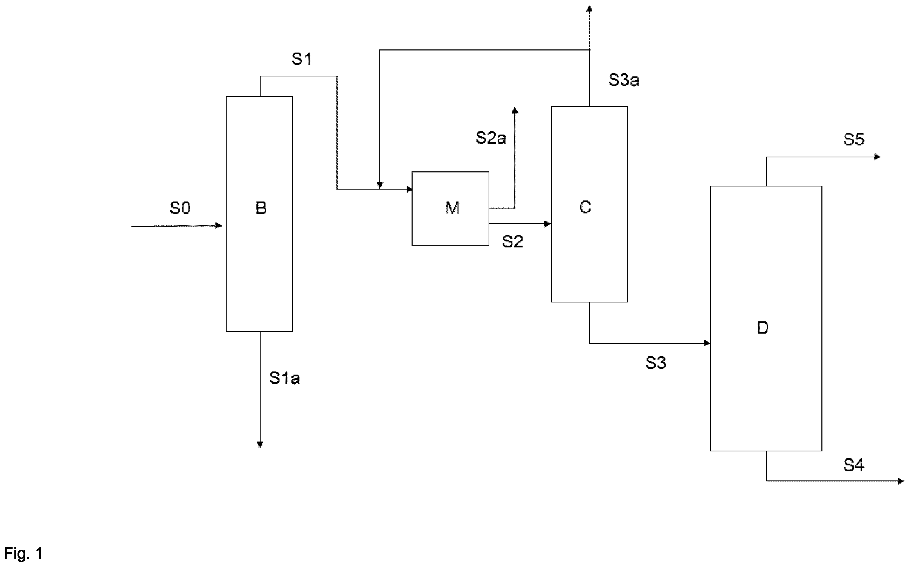

Fig. 1    shows the process for separating 1-methoxy-2-propanol and the involved columns B to D and membrane unit M schematically, without any heat integration. Stream S3a comprising water 1-methoxypropan-2-ol and 2-methoxypropan-1-ol is at least partially recycled to the membrane unit M, wherein the dotted line for stream S3a represents a non-recycled part, which might be absent in case of complete recycle of stream S3a.

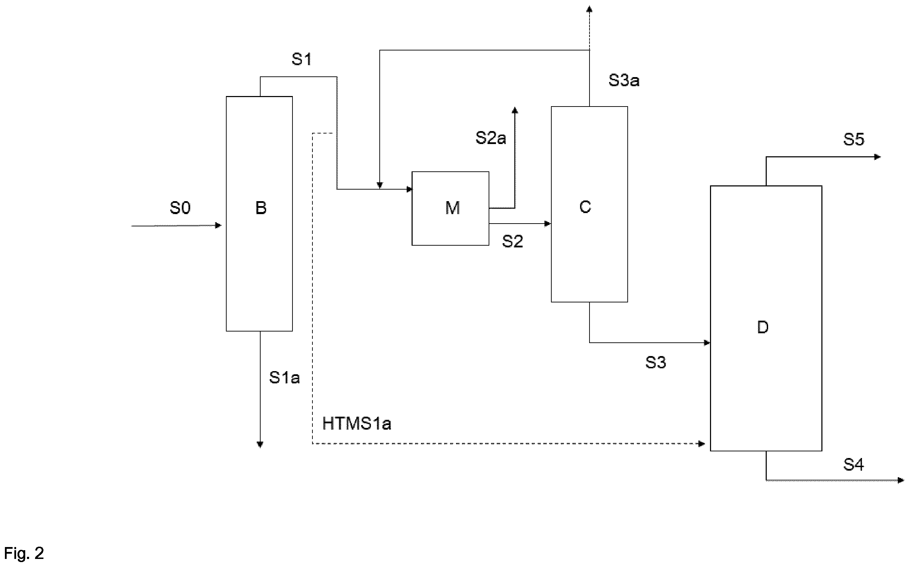

Fig. 2    shows the process for separating 1-methoxy-2-propanol and the involved columns B to D and distillation unit M schematically as in Fig. 1, together with a first option of heat integration, wherein the thermal energy (heat) of the condenser of column B, i.e. of stream S1, is used to heat the reboiler of column D in that thermal energy of stream S1 is partly transferred to a heat transfer medium stream HTMS1 in a heat exchanger H. The resulting heat transfer medium stream HTMS1a, which has an increased thermal energy content compared to HTMS1, is used to provide thermal energy to a heat exchanger unit, which supplies the reboiler of column D.

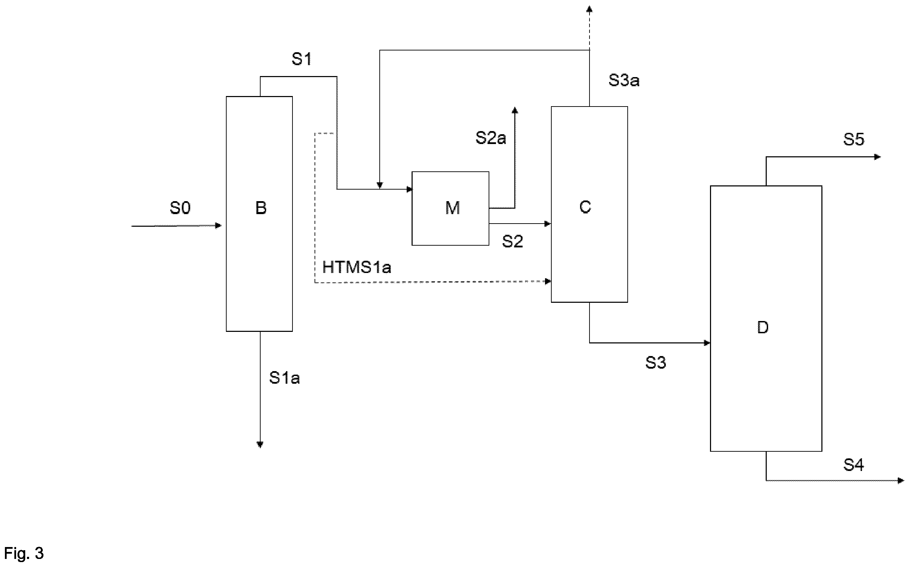

Fig. 3    shows the process for separating 1-methoxy-2-propanol and the involved columns B to D and membrane unit M schematically as in Fig. 1, together with a second option of heat integration, wherein the thermal energy (heat) of the condenser of column B, i.e. of stream S1, is used to heat the reboiler of column C in that thermal energy of stream S1 is partly transferred to a heat transfer medium stream HTMS1 in a heat exchanger H. The resulting heat transfer medium stream HTMS1a, which has an increased thermal energy content compared to HTMS1, is used to provide thermal energy to a heat exchanger unit, which supplies the reboiler of column C.

Fig. 4    shows the process for separating 1-methoxy-2-propanol and the involved columns B to D and membrane unit M schematically as in Fig. 1, together with a third option of heat integration, wherein the thermal energy (heat) of the condenser of column B, i.e. of stream S1, is used to heat the reboiler of membrane unit M and the reboiler of column C in that thermal energy of stream S1 is partly transferred to a heat transfer medium stream HTMS1 in a heat exchanger H. The resulting heat transfer medium stream HTMS1a, which has an increased thermal energy content compared to HTMS1, is used to provide thermal energy to a heat exchanger unit, which supplies the heat exchanger of membrane unit M and the reboiler of column C.

Fig. 5    shows the process for separating 1-methoxy-2-propanol and the involved columns B to D and membrane unit M schematically as in Fig. 1, together with a fourth, complete, heat integration. The thermal energy (heat) of the condenser of column B, i.e. of stream S1, is used to heat the reboiler of columns C and D and of the membrane unit M in that thermal energy of stream S1 is partly transferred to a heat transfer medium stream HTMS1 in a heat exchanger H, wherein a heat transfer medium stream HTMS1a is obtained which has an increased thermal energy content compared to HTMS1. The heat transfer medium stream HTMS1a is used to provide thermal energy to a heat exchanger unit, which supplies the reboiler of column C, to heat exchanger unit, which supplies the reboiler of column D, and to heat exchanger unit, which supplies the heat exchanger of membrane unit M.

# EP 4 237 399 B1

## Cited Literature

- US 5,723,024 A
- EP 1 375 462 A1
- EP 0 425 893 A
- DE 10 233 388 A1
- US 2004/0000473 A1
- CN 103342631 A
- CN 103992214 A

## Claims

1. A process for separating 1-methoxypropan-2-ol from an aqueous stream comprising 1-methoxypropan-2-ol and 2-methoxypropan-1-ol, wherein the process comprises:

   (a) Providing a stream S0 comprising 1-methoxypropan-2-ol, 2-methoxypropan-1-ol and water, and having a molar ratio of 1-methoxypropan-2-ol : 2-methoxypropan-1-ol in the range of from 1 : 5 to 5 : 1;
   (b) separating 1-methoxypropan-2-ol and 2-methoxypropan-1-ol from the stream S0 provided in (a) by distillation comprising subjecting the stream S0 provided in (a) to distillation conditions in a distillation unit comprising a distillation column B, obtaining a (top) stream S1 comprising 1-methoxypropan-2-ol, 2-methoxypropan-1-ol and water, which is enriched in 1-methoxypropan-2-ol and 2-methoxypropan-1-ol compared to the stream S0 and a bottoms stream S1a comprising water and being depleted of 1-methoxypropan-2-ol and 2-methoxypropan-1-ol compared to S0; wherein the distillation column B is operated at a pressure of $\geq 2$ bar;
   (c.1) separation of the stream S1 obtained in (b) with at least one membrane unit M comprising at least one membrane module, obtaining a stream S2 which is depleted of water and further enriched in 1-methoxypropan-2-ol and 2-methoxypropan-1-ol compared to the stream S1, and a stream S2a comprising water;
   (c.2) subjecting the stream S2 obtained in (c.1) to distillation conditions in a distillation unit comprising a distillation column C, obtaining a stream S3, which is depleted of water and further enriched in 1-methoxypropan-2-ol and 2-methoxypropan-1-ol compared to the stream S2, and a stream S3a comprising water;
   (d) separating 1-methoxypropan-2-ol from the stream S3 obtained in (c.2) by distillation, comprising subjecting the stream S2 obtained in (c.2) to distillation conditions in a distillation unit comprising a distillation column D, obtaining a stream S5 comprising $\geq 95$ weight-% 1-methoxypropan-2-ol and $\leq 0.5$ weight-% of 2-methoxypropan-1-ol, based on the total weight of stream S5, and a stream S4 comprising $\geq 95$ weight-% 2-methoxypropan-1-ol based on the total weight of stream S4;
   (e) optionally recirculating at least a part of the stream S3a to (c.1).

2. The process for separating 1-methoxypropan-2-ol from an aqueous stream comprising 1-methoxypropan-2-ol and 2-methoxypropan-1-ol according to claim 1, wherein the thermal energy of stream S1 is partly transferred to a heat transfer medium stream HTMS1 after (b) and before step (c), preferably in a heat exchanger H, obtaining a heat transfer medium stream HTMS1a having an increased thermal energy content compared to HTMS1;

   - wherein HTMS1a is used to provide thermal energy to:
   - the membrane unit of (c.1), preferably to a heat exchanger of the membrane unit of (c.1);
   and/or
   - the distillation unit of step (c.2), preferably to a heat exchanger unit connected to the distillation column C of the distillation unit of (c.2),
   and/or
   - the distillation unit of (d), preferably to a heat exchanger unit connected to the distillation column of the distillation unit of (d).

3. The process for separating 1-methoxypropan-2-ol from an aqueous stream comprising 1-methoxypropan-2-ol and 2-methoxypropan-1-ol according to claim 2, wherein the thermal energy provided by HTMS1a provides at least 90 % of the energy demand of the membrane unit of (c.1), and/or of the distillation unit of (c.2),and/or of the distillation unit of (d), preferably the thermal energy provided by HTMS1a provides at least 95 % of the energy demand of the membrane unit of (c.1), and/or of the distillation unit of (c.2),and/or of the distillation unit of (d); more preferably the thermal energy provided by HTMS1a provides at least 98 %, more preferably at least 99 %, more preferably 100 %, of the energy demand of the membrane unit of (c.1), and/or of the distillation unit of (c.2),and/or of the distillation unit of (d).

4. The process for separating 1-methoxypropan-2-ol from an aqueous stream comprising 1-methoxypropan-2-ol and 2-methoxypropan-1-ol according to claim 2 or 3, wherein in the range of from 40 to 95% of the thermal energy of stream S1 is transferred to the heat transfer medium stream HTMS1 obtaining a heat transfer medium stream HTMS1a having an increased thermal energy content compared to HTMS1.

5. The process for separating 1-methoxypropan-2-ol from an aqueous stream comprising 1-methoxypropan-2-ol and 2-methoxypropan-1-ol according to any one of claims 1 to 4, wherein stream S0 provided in a) comprises water in an amount in the range of from 50 to 90 weight-%, preferably in the range of from 55 to 85 weight-% and a mixture of 1-methoxypropan-2-ol and 2-methoxypropan-1-ol in an amount in the range of from 8 to 50 weight-%, preferably in the range of from 13 to 45 weight-%, each based on the total weight of stream S0, the remaining amount up to 100 weight-% being other components (impurities and solvent (MeOH)).

6. The process for separating 1-methoxypropan-2-ol from an aqueous stream comprising 1-methoxypropan-2-ol and 2-methoxypropan-1-ol according to any one of claims 1 to 5, wherein stream S0 provided in a) comprises 1-methoxypropan-2-ol and 2-methoxypropan-1 -ol in a molar ratio in the range of from 1 : 4 to 4 : 1; preferably in the range of from 1 : 3 to 3 : 1, more preferred in the range of from 1 : 2 to 2 : 1, more preferred in the range of from 0.75 : 1 to 1 : 0.75.

7. The process for separating 1-methoxypropan-2-ol from an aqueous stream comprising 1-methoxypropan-2-ol and 2-methoxypropan-1-ol according to any one of claims 1 to 6, wherein stream S0 provided in a) comprises propylene glycol dimethyl ether in an amount ≥ 0.001 weight-%, preferably in the range of from 0.001 to 0.1 weight-%, more preferred in the range of from 0.003 to 0.01 weight-%, more preferred in the range of from 0.004 to 0.01 weight-%, more preferred in the range of from 0.005 to 0.008 weight-%, based on the total weight of S0.

8. The process for separating 1-methoxypropan-2-ol from an aqueous stream comprising 1-methoxypropan-2-ol and 2-methoxypropan-1-ol according to any one of claims 1 to 7, wherein the distillation column B comprised in the distillation unit according to b) is operated at a pressure in the range of from 2 to 30 bar, preferably in the range of from 2.5 to 20 bar, more preferred in the range of from 3 to 15 bar, more preferred in the range of from 3.5 to 14 bar, more preferred in the range of from 4 to 12 bar, more preferred in the range of from 5 to 11 bar.

9. The process for separating 1-methoxypropan-2-ol from an aqueous stream comprising 1-methoxypropan-2-ol and 2-methoxypropan-1-ol according to any one of claims 1 to 8, wherein stream S1, which leaves distillation column B over the top, contains less than 0.05 weight-%, preferably less than 0.005 weight-%, more preferred less than 0.004 weight-%, more preferred less than 0.002 weight-%, of propylene glycol dimethyl ether, based on the total weight of S1.

10. The process for separating 1-methoxypropan-2-ol from an aqueous stream comprising 1-methoxypropan-2-ol and 2-methoxypropan-1-ol according to any one of claims 1 to 9, wherein the membrane unit M according to (c.1) comprises one or more membrane modules, each membrane module comprising at least one membrane, and optionally one or more further component selected from the group consisting of heat exchanger, pump compressor and condenser.

11. The process for separating 1-methoxypropan-2-ol from an aqueous stream comprising 1-methoxypropan-2-ol and 2-methoxypropan-1-ol according to any one of claims 1 to 10, wherein in step (e) at least 50 weight-% of S3a, preferably at least 60 weight-% of S3a, more preferred at least 70 weight-% of S3a, more preferred at least 80 weight-% of S3a, more preferred at least 90 weight-% of S3a, more preferred at least 95 weight-% of S3a, more preferred at least 99 weight-% of S3a, more preferred at least 99.5 weight-% of S3a, more preferred at least 100 weight-% of S3a, are recycled to the membrane unit M of (c.1).

12. The process for separating 1-methoxypropan-2-ol from an aqueous stream comprising 1-methoxypropan-2-ol and 2-methoxypropan-1-ol according to any one of claims 1 to 11, wherein the stream S4 is removed as bottoms stream from distillation column D, wherein S4 comprises ≥ 95 weight-% 2-methoxypropan-1-ol and ≤ 0.5 weight- % 1-methoxypropan-2-ol, preferably ≥ 96 weight-% 2-methoxypropan-1-ol and ≤ 0.1 weight- % 1-methoxypropan-2-ol, more preferred ≥ 98 weight-% 2-methoxypropan-1-ol and ≤ 0.001 weight- % 1-methoxypropan-2-ol, more preferred ≥ 99 weight-% 2-methoxypropan-1-ol and ≤ 0.0001 weight- % 1-methoxypropan-2-ol, each based on the total weight of stream S4.

13. The process for separating 1-methoxypropan-2-ol from an aqueous stream comprising 1-methoxypropan-2-ol and 2-methoxypropan-1-ol according to any one of claims 1 to 12, wherein the stream S5 is removed as top stream from distillation column D, which comprises ≥ 95 weight-% 1-methoxypropan-2-ol and ≤ 0.5 weight- % of 2-methoxypropan-1-ol, preferably ≥ 98 weight-% 1-methoxypropan-2-ol and ≤ 0.15 weight- % of 2-methoxypropan-1-ol, more preferred ≥ 99 weight-% 1-methoxypropan-2-ol and ≤ 0.1 weight- % of 2-methoxypropan-1-ol, each based on the total weight of stream S5.

14. The process for separating 1-methoxypropan-2-ol from an aqueous stream comprising 1-methoxypropan-2-ol and 2-methoxypropan-1-ol according to any one of claims 1 to 13, wherein the stream S5 comprises ≤ 0.01 weight-%, preferably ≤ 0.008 weight-%, more preferred ≤ 0.007 weight-%, more preferred ≤ 0.006 weight-%, of propylene glycol dimethyl ether based on the total weight of stream S5.


**Patentansprüche**

1. Verfahren zum Abtrennen von 1-Methoxypropan-2-ol aus einem wässrigen Strom umfassend 1-Methoxypropan-2-ol und 2-Methoxypropan-1-ol, wobei das Verfahren umfasst:

   (a) Bereitstellen eines Stroms S0 umfassend 1-Methoxypropan-2-ol, 2-Methoxypropan-1-ol und Wasser und mit einem Molverhältnis von 1-Methoxypropan-2-ol : 2-Methoxypropan-1-ol in dem Bereich von 1 : 5 bis 5 : 1;
   (b) Abtrennen von 1-Methoxypropan-2-ol und 2-Methoxypropan-1-ol aus dem bei (a) bereitgestellten Strom S0 durch Destillation, umfassend Unterwerfen des bei (a) bereitgestellten Stroms S0 an Destillationsbedingungen in einer Destillationseinheit umfassend eine Destillationskolonne B, um einen (Kopf-) Strom S1 umfassend 1-Methoxypropan-2-ol, 2-Methoxypropan-1-ol und Wasser, der im Vergleich zu dem Strom S0 an 1-Methoxypropan-2-ol und 2-Methoxypropan-1-ol angereichert ist, und einen Sumpfstrom S1a umfassend Wasser, der im Vergleich zu S0 an 1-Methoxypropan-2-ol und 2-Methoxypropan-1-ol abgereichert ist, zu erhalten; wobei die Destillationskolonne B bei einem Druck von ≥ 2 bar betrieben wird;
   (c.1) Auftrennen des bei (b) erhaltenen Stroms S1 mit wenigstens einer Membraneinheit M, die wenigstens ein Membranmodul umfasst, um einen Strom S2, der im Vergleich zu dem Strom S1 an Wasser abgereichert und an 1-Methoxypropan-2-ol und 2-Methoxypropan-1-ol weiter angereichert ist; und einen Strom S2a, der Wasser umfasst, zu erhalten;
   (c.2) Unterwerfen des bei (c.1) erhaltenen Stroms S2 an Destillationsbedingungen in einer Destillationseinheit umfassend eine Destillationskolonne C, um einen Strom S3, der im Vergleich zu dem Strom S2 an Wasser abgereichert und an 1-Methoxypropan-2-ol und 2-Methoxypropan-1-ol weiter angereichert ist, und einen Strom S3a, der Wasser umfasst, zu erhalten;
   (d) Abtrennen von 1-Methoxypropan-2-ol von dem bei (c.2) erhaltenen Strom S3 durch Destillation, umfassend Unterwerfen des bei (c.2) erhaltenen Stroms S2 an Destillationsbedingungen in einer Destillationseinheit umfassend eine Destillationskolonne D, um einen Strom S5 umfassend ≥ 95 Gew.-% 1-Methoxypropan-2-ol und ≤ 0,5 Gew.-% 2-Methoxypropan-1-ol, bezogen auf das Gesamtgewicht von Strom S5, und einen Strom S4 umfassend ≥ 95 Gew.-% 2-Methoxypropan-1-ol, bezogen auf das Gesamtgewicht von Strom S4, zu erhalten;
   (e) gegebenenfalls Rezirkulieren wenigstens eines Teils des Stroms S3a zu (c.1).

2. Verfahren zum Abtrennen von 1-Methoxypropan-2-ol aus einem wässrigen Strom umfassend 1-Methoxypropan-2-ol und 2-Methoxypropan-1-ol gemäß Anspruch 1, wobei die Wärmeenergie von Strom S1 nach (b) und vor Schritt (c) teilweise zu einem Wärmeträgermediumstrom HTMS1 überführt wird, vorzugsweise in einem Wärmetauscher H, um einen Wärmeträgermediumstrom HTMS1a mit einem im Vergleich zu HTMS1 erhöhten Wärmeenergieinhalt zu erhalten;

   - wobei HTMS1 a verwendet wird, um Wärmeenergie bereitzustellen an:
   - die Membraneinheit von (c.1), vorzugsweise an einen Wärmetauscher der Membraneinheit von (c.1): und/oder
   - die Destillationseinheit von Schritt (c.2), vorzugsweise an eine Wärmetauschereinheit, die mit der Destillationskolonne C der Destillationseinheit von (c.2) verbunden ist, und/oder
   - die Destillationseinheit von (d), vorzugsweise an eine Wärmetauschereinheit, die mit der Destillationskolonne der Destillationseinheit von (d) verbunden ist.

3. Verfahren zum Abtrennen von 1-Methoxypropan-2-ol aus einem wässrigen Strom umfassend 1-Methoxypropan-2-ol und 2-Methoxypropan-1-ol gemäß Anspruch 2, wobei die von HTMS1a bereitgestellte Wärmeenergie wenigstens 90 % des Energiebedarfs der Membraneinheit von (c.1) und/oder der Destillationseinheit von (c.2) und/oder der Destillationseinheit von (d) bereitstellt, vorzugsweise wobei die von HTMS1a bereitgestellte Wärmeenergie wenigstens 95 % des Energiebedarfs der Membraneinheit von (c.1) und/oder der Destillationseinheit von (c.2) und/oder der Destillationseinheit von (d) bereitstellt; bevorzugter wobei die von HTMS1a bereitgestellte Wärmeenergie wenigstens 98 %, bevorzugter wenigstens 99 %, bevorzugter 100 %, des Energiebedarfs der Membraneinheit von (c.1) und/oder der Destillationseinheit von (c.2) und/oder der Destillationseinheit von (d) bereitstellt.

4. Verfahren zum Abtrennen von 1-Methoxypropan-2-ol aus einem wässrigen Strom umfassend 1-Methoxypropan-2-ol und 2-Methoxypropan-1-ol gemäß Anspruch 2 oder 3, wobei in dem Bereich von 40 bis 95 % der Wärmeenergie von Strom S1 zu dem Wärmeträgermediumstrom HTMS1 überführt wird, um einen Wärmeträgermediumstrom HTMS1a mit einem im Vergleich zu HTMS1 erhöhten Wärmeenergieinhalt zu erhalten.

5. Verfahren zum Abtrennen von 1-Methoxypropan-2-ol aus einem wässrigen Strom umfassend 1-Methoxypropan-2-ol und 2-Methoxypropan-1-ol gemäß einem der Ansprüche 1 bis 4, wobei der bei a) bereitgestellte Strom S0 Wasser in einer Menge in dem Bereich von 50 bis 90 Gew.-%, vorzugsweise in dem Bereich von 55 bis 85 Gew.-%, und ein Gemisch von 1-Methoxypropan-2-ol und 2-Methoxypropan-1-ol in einer Menge in dem Bereich von 8 bis 50 Gew.-%, vorzugsweise in dem Bereich von 13 bis 45 Gew.-%, umfasst, jeweils bezogen auf das Gesamtgewicht von Strom S0, wobei die verbleibende Menge auf 100 Gew.-% andere Komponenten (Verunreinigungen und Lösungsmittel (MeOH)) sind.

6. Verfahren zum Abtrennen von 1-Methoxypropan-2-ol aus einem wässrigen Strom umfassend 1-Methoxypropan-2-ol und 2-Methoxypropan-1-ol gemäß einem der Ansprüche 1 bis 5, wobei der bei a) bereitgestellte Strom S0 1-Methoxypropan-2-ol und 2-Methoxypropan-1-ol in einem Molverhältnis in dem Bereich von 1 : 4 bis 4 : 1; vorzugsweise in dem Bereich von 1 : 3 bis 3 : 1, bevorzugter in dem Bereich von 1 : 2 bis 2 : 1, bevorzugter in dem Bereich von 0,75 : 1 bis 1 : 0,75, umfasst.

7. Verfahren zum Abtrennen von 1-Methoxypropan-2-ol aus einem wässrigen Strom umfassend 1-Methoxypropan-2-ol und 2-Methoxypropan-1-ol gemäß einem der Ansprüche 1 bis 6, wobei der bei a) bereitgestellte Strom S0 Propylenglycoldimethylether in einer Menge ≥ 0,001 Gew.-%, vorzugsweise in dem Bereich von 0,001 bis 0,1 Gew.-%, bevorzugter in dem Bereich von 0,003 bis 0,01 Gew.-%, bevorzugter in dem Bereich von 0,004 bis 0,01 Gew.-%, bevorzugter in dem Bereich von 0,005 bis 0,008 Gew.-%, bezogen auf das Gesamtgewicht von S0, umfasst.

8. Verfahren zum Abtrennen von 1-Methoxypropan-2-ol aus einem wässrigen Strom umfassend 1-Methoxypropan-2-ol und 2-Methoxypropan-1-ol gemäß einem der Ansprüche 1 bis 7, wobei die von der Destillationseinheit gemäß b) umfasste Destillationskolonne B bei einem Druck in dem Bereich von 2 bis 30 bar, vorzugsweise in dem Bereich von 2,5 bis 20 bar, bevorzugter in dem Bereich von 3 bis 15 bar, bevorzugter in dem Bereich von 3,5 bis 14 bar, bevorzugter in dem Bereich von 4 bis 12 bar, bevorzugter in dem Bereich von 5 bis 11 bar, betrieben wird.

9. Verfahren zum Abtrennen von 1-Methoxypropan-2-ol aus einem wässrigen Strom umfassend 1-Methoxypropan-2-ol und 2-Methoxypropan-1-ol gemäß einem der Ansprüche 1 bis 8, wobei der Strom S1, der aus der Destillationskolonne B über den Kopf austritt, weniger als 0,05 Gew.-%, vorzugsweise weniger als 0,005 Gew.-%, bevorzugter weniger als 0,004 Gew.-%, bevorzugter weniger als 0,002 Gew.-%, Propylenglycoldimethylether, bezogen auf das Gesamtgewicht von S1, enthält.

10. Verfahren zum Abtrennen von 1-Methoxypropan-2-ol aus einem wässrigen Strom umfassend 1-Methoxypropan-2-ol und 2-Methoxypropan-1-ol gemäß einem der Ansprüche 1 bis 9, wobei die Membraneinheit M gemäß (c.1) einen oder mehrere Membranmodule umfasst, wobei jedes Membranmodul wenigstens eine Membran und gegebenenfalls eine oder mehrere weitere Komponenten ausgewählt aus der Gruppe bestehend aus Wärmetauscher, Pumpenkompressor und Kondensator umfasst.

11. Verfahren zum Abtrennen von 1-Methoxypropan-2-ol aus einem wässrigen Strom umfassend 1-Methoxypropan-2-ol und 2-Methoxypropan-1-ol gemäß einem der Ansprüche 1 bis 10, wobei bei Schritt (e) wenigstens 50 Gew.-% von S3a, vorzugsweise wenigstens 60 Gew.-% von S3a, bevorzugter wenigstens 70 Gew.-% von S3a, bevorzugter wenigstens 80 Gew.-% von S3a, bevorzugter wenigstens 90 Gew.-% von S3a, bevorzugter wenigstens 95 Gew.-

% von S3a, bevorzugter wenigstens 99 Gew.-% von S3a, bevorzugter wenigstens 99,5 Gew.-% von S3a, bevorzugter wenigstens 100 Gew.-% von S3a, zu der Membraneinheit M von (c.1) zurückgeführt werden.

12. Verfahren zum Abtrennen von 1-Methoxypropan-2-ol aus einem wässrigen Strom umfassend 1-Methoxypropan-2-ol und 2-Methoxypropan-1-ol gemäß einem der Ansprüche 1 bis 11, wobei der Strom S4 als Sumpfstrom aus der Destillationskolonne D entnommen wird, wobei S4 ≥ 95 Gew.-% 2-Methoxypropan-1-ol und ≤ 0,5 Gew.-% 1-Methoxypropan-2-ol, vorzugsweise ≥ 96 Gew.-% 2-Methoxypropan-1-ol und ≤ 0,1 Gew.-% 1-Methoxypropan-2-ol, bevorzugter ≥ 98 Gew.-% 2-Methoxypropan-1-ol und ≤ 0,001 Gew.-% 1-Methoxypropan-2-ol, bevorzugter ≥ 99 Gew.-% 2-Methoxypropan-1-ol und ≤ 0,0001 Gew.-% 1-Methoxypropan-2-ol, jeweils bezogen auf das Gesamtgewicht von Strom S4, umfasst.

13. Verfahren zum Abtrennen von 1-Methoxypropan-2-ol aus einem wässrigen Strom umfassend 1-Methoxypropan-2-ol und 2-Methoxypropan-1-ol gemäß einem der Ansprüche 1 bis 12, wobei der Strom S5 als Kopfstrom aus der Destillationskolonne D entnommen wird, der ≥ 95 Gew.-% 1-Methoxypropan-2-ol und ≤ 0,5 Gew.-% 2-Methoxypropan-1-ol, vorzugsweise ≥ 98 Gew.-% 1-Methoxypropan-2-ol und ≤ 0,15 Gew.-% 2-Methoxypropan-1-ol, bevorzugter ≥ 99 Gew.-% 1-Methoxypropan-2-ol und ≤ 0,1 Gew.-% 2-Methoxypropan-1-ol, jeweils bezogen auf das Gesamtgewicht von Strom S5, umfasst.

14. Verfahren zum Abtrennen von 1-Methoxypropan-2-ol aus einem wässrigen Strom umfassend 1-Methoxypropan-2-ol und 2-Methoxypropan-1-ol gemäß einem der Ansprüche 1 bis 13, wobei der Strom S5 ≤ 0,01 Gew.-%, vorzugsweise ≤ 0,008 Gew.-%, bevorzugter ≤ 0,007 Gew.-%, bevorzugter ≤ 0,006 Gew.-%, Propylenglycoldimethylether, bezogen auf das Gesamtgewicht von Strom S5, umfasst.

## Revendications

1. Procédé de séparation de 1-méthoxypropan-2-ol d'un flux aqueux comprenant du 1-méthoxypropan-2-ol et du 2-méthoxypropan-1-ol, le procédé comprenant :

   (a) la fourniture d'un flux S0 comprenant du 1-méthoxypropan-2-ol, du 2-méthoxypropan-1-ol et de l'eau, et ayant un rapport molaire de 1-méthoxypropan-2-ol : 2-méthoxypropan-1-ol dans la plage de 1 : 5 à 5 : 1 ;
   (b) la séparation du 1-méthoxypropan-2-ol et du 2-méthoxypropan-1-ol du flux S0 fourni en (a) par distillation comprenant la soumission du flux S0 fourni en (a) à des conditions de distillation dans une unité de distillation comprenant un colonne de distillation B, de façon à obtenir un flux (de tête) S1 comprenant du 1-méthoxypropan-2-ol, du 2-méthoxypropan-1-ol et de l'eau, qui est enrichi en 1-méthoxypropan-2-ol et en 2-méthoxypropan-1-ol par rapport au flux S0 et un flux de queue S1a comprenant de l'eau et étant appauvri en 1-méthoxypropan-2-ol et en 2-méthoxypropan-1-ol par rapport à S0 ; la colonne de distillation B fonctionnant à une pression ≥ 2 bar ;
   (c.1) la séparation du flux S1 obtenu en (b) avec au moins une unité à membrane M comprenant au moins un module à membrane, de façon à obtenir un flux S2 qui est appauvri en eau et enrichi davantage en 1-méthoxypropan-2-ol et en 2-méthoxypropan-1-ol par rapport au flux S1, et un flux S2a comprenant de l'eau ;
   (c.2) la soumission du flux S2 obtenu en (c.1) à des conditions de distillation dans une unité de distillation comprenant une colonne de distillation C, de façon à obtenir un flux S3 appauvri en eau et enrichi davantage en 1-méthoxypropan-2-ol et en 2-méthoxypropan-1-ol par rapport au flux S2, et un flux S3a comprenant de l'eau ;
   (d) la séparation du 1-méthoxypropan-2-ol du flux S3 obtenu en (c.2) par distillation, comprenant la soumission du flux S2 obtenu en (c.2) à des conditions de distillation dans une unité de distillation comprenant une colonne de distillation D, de façon à obtenir un flux S5 comprenant ≥ 95 % en poids de 1-méthoxypropan-2-ol et ≤ 0,5 % en poids de 2-méthoxypropan-1-ol, par rapport au poids total du flux S5, et un flux S4 comprenant ≥ 95 % en poids de 2-méthoxypropan-1-ol par rapport au poids total du flux S4 ;
   (e) éventuellement la recirculation d'au moins une partie du flux S3a vers (c.1).

2. Procédé de séparation de 1-méthoxypropan-2-ol d'un flux aqueux comprenant du 1-méthoxypropan-2-ol et du 2-méthoxypropan-1-ol selon la revendication 1, dans lequel l'énergie thermique du flux S1 est partiellement transférée à un flux de fluide caloporteur HTMS1 après (b) et avant l'étape (c), de préférence dans un échangeur de chaleur H, de façon à obtenir un flux de fluide caloporteur HTMS1a ayant une teneur en énergie thermique accrue par rapport à HTMS1 ;

   - dans lequel HTMS1a est utilisé pour fournir de l'énergie thermique à :
   - l'unité à membrane de (c.1), de préférence à un échangeur de chaleur de l'unité à membrane de (c.1) ; et/ou

- l'unité de distillation de l'étape (c.2), de préférence à une unité d'échangeur de chaleur reliée à la colonne de distillation C de l'unité de distillation de (c.2),
et/ou
- l'unité de distillation de (d), de préférence à une unité d'échangeur de chaleur reliée à la colonne de distillation de l'unité de distillation de (d).

3. Procédé de séparation de 1-méthoxypropan-2-ol d'un flux aqueux comprenant du 1-méthoxypropan-2-ol et du 2-méthoxypropan-1-ol selon la revendication 2, dans lequel l'énergie thermique délivrée par HTMS1a fournit au moins 90 % de la demande énergétique de l'unité à membrane de (c.1), et/ou de l'unité de distillation de (c.2), et/ou de l'unité de distillation de (d), de préférence l'énergie thermique délivrée par HTMS1a fournit au moins 95 % de la demande énergétique de l'unité à membrane de (c.1), et/ou de l'unité de distillation de (c.2), et/ou de l'unité de distillation de (d) ; plus préférablement, l'énergie thermique délivrée par HTMS1a fournit au moins 98 %, plus préférablement au moins 99 %, plus préférablement 100 %, de la demande énergétique de l'unité à membrane de (c.1), et/ou de l'unité de distillation de (c.2), et/ou de l'unité de distillation de (d).

4. Procédé de séparation de 1-méthoxypropan-2-ol d'un flux aqueux comprenant du 1-méthoxypropan-2-ol et du 2-méthoxypropan-1-ol selon la revendication 2 ou 3, dans lequel 40 à 95 % du l'énergie thermique du flux S1 est transférée au flux de fluide caloporteur HTMS1, de façon à obtenir un flux de fluide caloporteur HTMS1a ayant une teneur en énergie thermique accrue par rapport à HTMS1.

5. Procédé de séparation de 1-méthoxypropan-2-ol d'un flux aqueux comprenant du 1-méthoxypropan-2-ol et du 2-méthoxypropan-1-ol selon l'une quelconque des revendications 1 à 4, dans lequel le flux S0 fourni en a) comprend de l'eau en une quantité dans la plage de 50 à 90 % en poids, de préférence dans la plage de 55 à 85 % en poids et un mélange de 1-méthoxypropan-2-ol et de 2-méthoxypropan-1-ol en une quantité dans la plage de 8 à 50 % en poids, de préférence dans la plage de 13 à 45 % en poids, chacun par rapport au poids total du flux S0, le complément à 100 % en poids étant constituée d'autres composants (impuretés et solvant (MeOH)).

6. Procédé de séparation de 1-méthoxypropan-2-ol d'un flux aqueux comprenant du 1-méthoxypropan-2-ol et du 2-méthoxypropan-1-ol selon l'une quelconque des revendications 1 à 5, dans lequel le flux S0 fourni en a) comprend du 1-méthoxypropan-2-ol et du 2-méthoxypropan-1-ol dans un rapport molaire dans la plage de 1 : 4 à 4 : 1 ; de préférence dans la plage de 1 : 3 à 3 : 1, plus préférablement dans la plage de 1 : 2 à 2 : 1, plus préférablement dans la plage de 0,75 : 1 à 1 : 0,75.

7. Procédé de séparation de 1-méthoxypropan-2-ol d'un flux aqueux comprenant du 1-méthoxypropan-2-ol et du 2-méthoxypropan-1-ol selon l'une quelconque des revendications 1 à 6, dans lequel le flux S0 fourni en a) comprend de l'éther diméthylique de propylène glycol en une quantité $\geq$ 0,001 % en poids, de préférence dans la plage de 0,001 à 0,1 % en poids, plus préférablement dans la plage de 0,003 à 0,01 % en poids, plus préférablement dans la plage de 0,004 à 0,01 % en poids, plus préférablement dans la plage de 0,005 à 0,008 % en poids, par rapport au poids total de S0.

8. Procédé de séparation de 1-méthoxypropan-2-ol d'un flux aqueux comprenant du 1-méthoxypropan-2-ol et du 2-méthoxypropan-1-ol selon l'une quelconque des revendications 1 à 7, dans lequel la colonne de distillation B comprise dans l'unité de distillation selon b) fonctionne à une pression dans la plage de 2 à 30 bar, de préférence dans la plage de 2,5 à 20 bar, plus préférablement dans la plage de 3 à 15 bar, plus préférablement dans la plage de 3,5 à 14 bar, plus préférablement dans la plage de 4 à 12 bar, plus préférablement dans la plage de 5 à 11 bar.

9. Procédé de séparation de 1-méthoxypropan-2-ol d'un flux aqueux comprenant du 1-méthoxypropan-2-ol et du 2-méthoxypropan-1-ol selon l'une quelconque des revendications 1 à 8, dans lequel le flux S1, qui sort de la colonne de distillation B par le haut, contient moins de 0,05 % en poids, de préférence moins de 0,005 % en poids, plus préférablement moins de 0,004 % en poids, plus préférablement moins de 0,002 % en poids, d'éther diméthylique de propylène glycol, par rapport au poids total de S1.

10. Procédé de séparation de 1-méthoxypropan-2-ol d'un flux aqueux comprenant du 1-méthoxypropan-2-ol et du 2-méthoxypropan-1-ol selon l'une quelconque des revendications 1 à 9, dans lequel l'unité à membrane M selon (c.1) comprend un ou plusieurs modules à membrane, chaque module à membrane comprenant au moins une membrane, et éventuellement un ou plusieurs autres composants choisis dans le groupe constitué d'un échangeur de chaleur, d'un compresseur à pompe et d'un condenseur.

**11.** Procédé de séparation de 1-méthoxypropan-2-ol d'un courant aqueux comprenant du 1-méthoxypropan-2-ol et du 2-méthoxypropan-1-ol selon l'une quelconque des revendications 1 à 10, dans lequel, à l'étape (e), au moins 50 en poids de S3a, de préférence au moins 60 % en poids de S3a, plus préférablement au moins 70 % en poids de S3a, plus préférablement au moins 80 % en poids de S3a, plus préférablement au moins 90 % en poids de S3a, plus de préférence, au moins 95 % en poids de S3a, plus préférablement au moins 99 % en poids de S3a, plus préférablement au moins 99,5 % en poids de S3a, plus préférablement au moins 100 % en poids de S3a, sont recyclés vers l'unité à membrane M de (c.1).

**12.** Procédé de séparation de 1-méthoxypropan-2-ol d'un flux aqueux comprenant du 1-méthoxypropan-2-ol et du 2-méthoxypropan-1-ol selon l'une quelconque des revendications 1 à 11, dans lequel le flux S4 est éliminé en tant que flux de queue provenant de la colonne de distillation D, dans lequel S4 comprend ≥ 95 % en poids de 2-méthoxypropan-1-ol et ≤ 0,5 % en poids de 1-méthoxypropan-2-ol, de préférence ≥ 96 % en poids de 2-méthoxypropan-1-ol et ≤ 0,1 % en poids de 1-méthoxypropan-2-ol, plus préférablement ≥ 98 % en poids de 2-méthoxypropan-1-ol et ≤ 0,001 % en poids de 1-méthoxypropan-2-ol, plus préférablement ≥ 99 % en poids de 2-méthoxypropan-1-ol et ≤ 0,0001 % en poids de 1-méthoxypropan-2-ol, chacun par rapport au poids total du flux S4.

**13.** Procédé de séparation de 1-méthoxypropan-2-ol d'un flux aqueux comprenant du 1-méthoxypropan-2-ol et du 2-méthoxypropan-1-ol selon l'une quelconque des revendications 1 à 12, dans lequel le flux S5 est éliminé en tant que flux supérieur provenant de la colonne de distillation D, qui comprend ≥ 95 % en poids de 1-méthoxypropan-2-ol et ≤ 0,5 % en poids de 2-méthoxypropan-1-ol, de préférence ≥ 98 % en poids de 1-méthoxypropan-2-ol et ≤ 0,15 % en poids de 2-méthoxypropan-1-ol, plus préférablement ≥ 99 % en poids de 1-méthoxypropan-2-ol et ≤ 0,1 % en poids de 2-méthoxypropan-1-ol, chacun par rapport au poids total du flux S5.

**14.** Procédé de séparation de 1-méthoxypropan-2-ol d'un flux aqueux comprenant du 1-méthoxypropan-2-ol et du 2-méthoxypropan-1-ol selon l'une quelconque des revendications 1 à 13, dans lequel le flux S5 comprend ≤ 0,01 % en poids, de préférence ≤ 0,008 % en poids, plus préférablement ≤ 0,007% en poids, plus préférablement < 0,006 % en poids d'éther diméthylique de propylène glycol par rapport au poids total du flux S5.

Fig. 1

Fig. 2

Fig. 3

EP 4 237 399 B1

Fig. 4

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5723024 A
- EP 0425893 A
- DE 10233388 A1
- US 20040000473 A1
- EP 1375462 A1
- CN 103342631 A
- CN 103992214 A